# EUROPEAN PATENT APPLICATION

(11) **EP 4 046 617 A1**
(43) Date of publication of application: **24.08.2022**
(21) Application number: 22159708.1
(22) Date of filing: 21.08.2019
(51) Int. Cl.: A61F 13/537, A61F 13/49, A61F 13/494, A61F 13/511, A61F 13/513, A61F 13/56

(54) **ABSORBENT ARTICLE**

(30) Priority: 21.08.2018 JP 2018154717; 21.08.2018 JP 2018154718
(62) Divisional of application: 19852236.9
(71) Applicant: Unicharm Corporation, Ehime 799-0111 (JP)
(72) Inventor: KAWAKAMI, Yusuke, Tokyo (JP); SHEN, Lianlian, Shanghai (CN)
(74) Representative: Staeger & Sperling Partnerschaftsgesellschaft mbB

(57) **Abstract**

An absorbent article (1) having an absorbent body, a skin-side sheet located on a skin side with respect to the absorbent body, and an intermediate sheet located between the absorbent body and the skin-side sheet, the skin-side sheet having a first region and a second region, the first region being a region that forms a protruded and recessed shape, the second region being a region that is adjacent to the first region on a front side in the longitudinal direction, the first region having a larger sparseness difference of fibers in a plane direction of the skin-side sheet than the second region has, a skin-side surface of the intermediate sheet being flat compared with the protruded and recessed shape of the first region, the skin-side sheet having a second joining region and a first joining region, the second joining region being a region where one part of the intermediate sheet is joined to the second region, the first joining region being a region where another part of the intermediate sheet is joined to the first region.

## Description

### [Technical Field]

The present invention relates to an absorbent article and a method for manufacturing an absorbent article.

### [Background Art]

Patent Document 1 discloses a disposable diaper in which an intermediate sheet is interposed between a top sheet and an absorbent body in order to prevent the backflow of urine absorbed by the absorbent body. In addition, in Patent Document 1, when the front end of the top sheet is assumed as 0%, and the back end of the top sheet is assumed as 100%, it is preferable that the front end of the intermediate sheet is positioned in a range of 0% to 11 % and the back end of the intermediate sheet is positioned in a range of 92% to 100%. The intermediate sheet is disposed in a broad range from the front portion through the back portion of the diaper.

Patent Document 2 discloses the following method for manufacturing a top-surface sheet of an absorbent article: a first roll has a protruded and recessed shape on the circumferential surface; a second roll has on the circumferential surface a protruded and recessed shape that is engaged with the protruded and recessed shape of the first roll; a sheet for an upper layer is sandwiched between the first roll and the second roll so as to shape protrusions and recesses; and a sheet for a lower layer is overlaid on and joined to the sheet for the upper layer in which protrusions and recesses have been shaped.

### [Patent Document]

[Patent Document 1] Japanese Patent Application Publication No. 2013-172813
[Patent Document 2] Japanese Patent Application Publication No. 2004-174234

### [Summary of the Invention]

### [Technical Problem]

A problem to be solved by the present invention is at least one of a first problem and a second problem below. The first problem is that, in disposable diapers, not only urine but also feces are excreted. Soft feces containing a large amount of moisture has a problem in that the soft feces is likely to leak from the back side and is likely to be attached to skin. As in the diaper of Patent Document 1, when the intermediate sheet is disposed up to the back portion of the diaper, moisture contained in soft feces rapidly transfers to the intermediate sheet, making it possible to suppress the flow of the soft feces. However, soft feces from which only moisture is first absorbed becomes more viscous, making it difficult to be taken into the inside of the top sheet. As a result, the soft feces is likely to remain on the surface of the top sheet (skin-side sheet), and the soft feces is likely to be attached to the skin of a wearer.

Therefore, an aspect of the present invention is to reduce the amount of soft feces that remains in the skin-side sheet, while suppressing the leakage of soft feces from the back side.

The second problem is that, in the case of shaping protrusions and recesses by passing a sheet (skin-side sheet) between a pair of rolls having a protruded and recessed shape on their circumferential surfaces, the sheet is stretched in multiple directions, causing the sparseness difference of fibers in the sheet. Therefore, in a portion of the sheet in which protrusions and recesses are shaped, a tensile force for transporting the sheet is unevenly applied, making it more likely to loosen the sheet. When another sheet (intermediate sheet) is joined in this state, there is a risk that wrinkles occur in the joining portion of the sheet.

Therefore, an aspect of the present invention is to make it less likely for wrinkles to occur in an absorbent article whose intermediate sheet is joined to a skin-side sheet having protrusions and recesses which are shaped by passing the skin-side sheet between a pair of rolls having protruding portions on their outer circumferential surfaces.

### [Solution to Problem]

A main aspect of the present disclosure for solving the first problem is an absorbent article having a longitudinal direction and a lateral direction,
the absorbent article including:
an absorbent body;
a skin-side sheet located on a skin side with respect to the absorbent body; and
an intermediate sheet located between the absorbent body and the skin-side sheet,
the skin-side sheet having a first surface region and a second surface region,
   the first surface region having a first surface shape,
   the second surface region having a second surface shape that is different from the first surface shape,
the first surface region having a arrangement region and a non-arrangement region,
   the arrangement region being a region that overlaps the intermediate sheet from the skin side,
   the non-arrangement region being a region that does not overlap the intermediate sheet, in a state where the absorbent article is unfolded and stretched,
   at least a part of the arrangement region being located on a back side with respect to a longitudinal center of the absorbent article,
   at least a part of the non-arrangement region being located on the back side in the longitudinal direction with respect to the arrangement region.

A main aspect of the present disclosure for solving the second problem is a method for manufacturing an absorbent article,
the absorbent article having
   an absorbent body,
   a skin-side sheet located on a skin side with respect to the absorbent body, and
   an intermediate sheet located between the absorbent body and the skin-side sheet, the method including:
a step of passing the skin-side sheet between a pair of rolls,
   shaping a first region of the skin-side sheet into a protruded and recessed shape and
   not shaping a second region of the skin-side sheet into a protruded and recessed shape,
   the pair of rolls having a protruding portion provided on an outer circumferential surface of at least either one of the pair of rolls,
   the second region being a region that is adjacent to the first region in a direction of transport of the skin-side sheet; and
a step of joining an intermediate sheet and the skin-side sheet,
   the intermediate sheet having a skin-side surface that is flat compared with the protruded and recessed shape of the first region,
   joining being performed in which one part of the intermediate sheet is joined to the second region of the skin-side sheet,
   subsequently, joining being performed in which another part of the intermediate sheet is joined to the first region of the skin-side sheet. Other features of the present invention will be further clarified by the description of the present specification and the accompanying drawings.

### [Advantageous Effect of the Invention]

An effect of the present disclosure on the first problem is that it is possible to reduce the amount of soft feces that remains in the skin-side sheet, while suppressing the leakage of soft feces from the back side.

An effect of the present disclosure on the second problem is that it is less likely for wrinkles to occur in an absorbent article whose intermediate sheet is joined to a skin-side sheet having protrusions and recesses which are shaped by passing the skin-side sheet between a pair of rolls having protruding portions on their outer circumferential surfaces.

### [Brief Description of Drawings]

FIG. 1 is a plan view of a diaper 1 in an unfolded and stretched state.
FIG. 2 is a cross-sectional view taken along a line A-A shown in FIG. 1.
FIG. 3A is a cross-sectional view of a second surface region 32 of a skin-side sheet 3, FIG. 3B is a cross-sectional view of a first surface region 31 of the skin-side sheet 3, and FIG. 3C is a diagram illustrating how to process the skin-side sheet 3.
FIG. 4 is a view showing the arrangement of the first surface region 31 and an intermediate sheet 4.
FIG. 5 is a view showing the arrangement of the first surface region 31 and a leak-proof wall portion 11.
FIGS. 6A and 6B are explanatory diagrams illustrating how to absorb urine.
FIGS. 7A to 7C are explanatory diagrams illustrating how to absorb soft feces.
FIGS. 8A to 8C are explanatory diagrams illustrating how to absorb soft feces.
FIG. 9 is a plan view of a diaper 100 in an unfolded and stretched state.
FIG. 10 is a cross-sectional view taken along a line A-A shown in FIG. 9.
FIG. 11 is a view schematically showing a part of a manufacturing apparatus 200 of the diaper 100.
FIG. 12 is an enlarged view of protrusion-and-recess-shaping portions 211 and 221.
FIG. 13A is a cross-sectional view of an intermediate sheet 104, FIG. 13B is a cross-sectional view of a first region 1031 of a skin-side sheet 103, and FIG. 13C is a cross-sectional view of a second region 1032 of the skin-side sheet 103.
FIG. 14 is a diagram illustrating a state where the intermediate sheets 104 are joined to the skin-side sheets 103 (continuous body).
FIGS. 15A to 15C are explanatory diagrams illustrating a folding step of the diaper 100.
FIG. 16 is a schematic plan view of the diaper 100 manufactured by the manufacturing apparatus 200 of the diaper 100.
FIG. 17 is a view showing the arrangement of the first region 1031 of the skin-side sheet 103 and a leak-proof wall portion 1011.
FIG. 18 is a schematic plan view of the diaper 100 of a modified example.
FIGS. 19A and 19B are explanatory diagrams illustrating how to absorb urine.
FIGS. 20A to 20C are explanatory diagrams illustrating how to absorb soft feces.
FIGS. 21A to 21C are explanatory diagrams illustrating how to absorb soft feces.

### [Description of Embodiments]

Descriptions in the present specification and the accompanying drawings clarify at least the following matters. An absorbent article having a longitudinal direction and a lateral direction,
the absorbent article including:
an absorbent body;
a skin-side sheet located on a skin side with respect to the absorbent body; and
an intermediate sheet located between the absorbent body and the skin-side sheet,
the skin-side sheet having a first surface region and a second surface region,
   the first surface region having a first surface shape,
   the second surface region having a second surface shape that is different from the first surface shape,
the first surface region having a arrangement region and a non-arrangement region,
   the arrangement region being a region that overlaps the intermediate sheet from the skin side,
   the non-arrangement region being a region that does not overlap the intermediate sheet,
in a state where the absorbent article is unfolded and stretched,
   at least a part of the arrangement region being located on a back side with respect to a longitudinal center of the absorbent article,
   at least a part of the non-arrangement region being located on the back side in the longitudinal direction with respect to the arrangement region.

According to the above-described absorbent article, moisture in soft feces in the arrangement region can be transferred to the intermediate sheet, making it possible to suppress the flow of the soft feces. Therefore, it is possible to suppress the leakage of soft feces from the back side. In addition, the entire soft feces that has flowed into the non-arrangement region on the back side beyond the arrangement region is more likely to be taken into the inside of the skin-side sheet, making it possible to reduce the amount of soft feces that remains on the skin-side sheet.

In such an absorbent article,
the first surface region has a larger sparseness difference of fibers in a plane direction of the skin-side sheet than the second surface region has.

According to the above-described absorbent article, it is easy to take all soft feces into the inside of the skin-side sheet from a portion of the first surface region where fibers are sparse, making it possible to reduce the amount of soft feces that remains on the skin-side sheet.

In such an absorbent article,
in the first surface region,
a space is provided between the skin-side sheet and a material that is adjacent to the skin-side sheet on a non-skin side.

According to the above-described absorbent article, it is easy to take a larger amount of soft feces into the space below (on non-skin side of) the skin-side sheet, making it possible to reduce the amount of soft feces that remains on the skin-side sheet.

In such an absorbent article,
in the first surface region,
the skin-side sheet has a protruded and recessed shape, and
a side wall portion of protrusions and recesses has a sparse density of fibers compared with an apex portion of the protrusions and recesses.

According to the above-described absorbent article, it is easy to take all soft feces into the inside of the skin-side sheet from the side wall portion (a portion where fibers are sparse), making it possible to reduce the amount of soft feces that remains on the skin-side sheet.

In such an absorbent article,
the second surface region has a second arrangement region that overlaps the intermediate sheet from the skin side, and,
in a state where the absorbent article is unfolded and stretched,
   at least a part of the second arrangement region is positioned on a front side in the longitudinal direction with respect to the arrangement region.

According to the above-described absorbent article, the sparseness difference of fibers is small in the second surface region, making it easier to transfer urine to the intermediate sheet across the entire region of the second arrangement region, and it is possible to suppress urine leakage.

In such an absorbent article,
in a state where the absorbent article is unfolded and stretched,
a longitudinal length of the second arrangement region is longer than a sum of a longitudinal length of the arrangement region and a longitudinal length of the non-arrangement region.

According to the above-described absorbent article, the second arrangement region suitable for urination can be disposed broadly in the longitudinal direction, making it possible to suppress urine leakage.

In such an absorbent article,
the absorbent article is a tape-type absorbent article having a pair of fastening tapes that extend from a back waist region toward two lateral outer sides, and
in a state where the absorbent article is unfolded and stretched,
   the first surface region is positioned on a front side in the longitudinal direction with respect to longitudinal centers of the fastening tapes.

According to the above-described absorbent article, the first surface region having a portion where fibers are dense (a highly stiff portion) is not brought by the fastening tapes into close contact with a wearer strongly, making it possible to enhance wearing comfortability.

In such an absorbent article,
the absorbent article is a tape-type absorbent article having a pair of fastening tapes that extend from a back waist region toward two lateral outer sides,
a waist stretchable member that stretches and contracts in the lateral direction is disposed in the back waist region, and
in a state where the absorbent article is unfolded and stretched,
   the first surface region is positioned on a front side in the longitudinal direction with respect to the waist stretchable member.

According to the above-described absorbent article, the first surface region having a portion where fibers are dense (a highly stiff portion) is not brought by the waist stretchable member into close contact with a wearer strongly, making it possible to enhance wearing comfortability.

In such an absorbent article,
in a state where the absorbent article is unfolded and stretched,
a longitudinal length of the non-arrangement region is longer than a longitudinal length of the arrangement region.

According to the above-described absorbent article, the non-arrangement region, in which soft feces is easy to be taken into the inside of the skin-side sheet, can be broadly disposed in the longitudinal direction, making it possible to reduce the amount of soft feces that remains on the skin-side sheet.

In such an absorbent article,
in the first surface region, a plurality of protruding portions that protrude toward the skin side are arranged side-by-side in the lateral direction.

According to the above-described absorbent article, the protruding portions makes it possible to suppress the flow of soft feces toward the back side in the longitudinal direction, making it possible to suppress the leakage of soft feces from the back side.

In such an absorbent article,
in the first surface region, the plurality of protruding portions are arranged side-by-side in the longitudinal direction.

According to the above-described absorbent article, the protruding portions makes it possible to suppress the flow of soft feces outward in the lateral direction, making it possible to suppress the leakage of soft feces from a leg circumference opening portion.

In such an absorbent article,
in a state where the absorbent article is unfolded and stretched,
a lateral length of the non-arrangement region is longer than a lateral length of the arrangement region.

According to the above-described absorbent article, soft feces that has spread in the lateral direction is more likely to be taken into the inside of the skin-side sheet in the non-arrangement region, making it possible to reduce the amount of soft feces that remains on the skin-side sheet.

In such an absorbent article,
the absorbent article further comprises a pair of leak-proof wall portions that rise up toward the skin side, in two lateral side portions of the absorbent body, and
in a state where the absorbent article is unfolded and stretched,
   at least a part of a portion of the non-arrangement region that extends in the lateral direction beyond the arrangement region is positioned on a central side in the lateral direction with respect to the leak-proof wall portions.

According to the above-described absorbent article, in the non-arrangement region between the arrangement region and the leak-proof wall portion in the lateral direction, soft feces can be guided to the inner side of the leak-proof wall portion while taking the soft feces into the inside of the skin-side sheet. Therefore, it is possible to suppress the leakage of soft feces from a leg circumference opening portion.

A method for manufacturing an absorbent article,
the absorbent article having
   an absorbent body,
   a skin-side sheet located on a skin side with respect to the absorbent body, and
   an intermediate sheet located between the absorbent body and the skin-side sheet,
the method including:
   a step of passing the skin-side sheet between a pair of rolls,
      shaping a first region of the skin-side sheet into a protruded and recessed shape and not shaping a second region of the skin-side sheet into a protruded and recessed shape,
      the pair of rolls having a protruding portion provided on an outer circumferential surface of at least either one of the pair of rolls,
      the second region being a region that is adjacent to the first region in a direction of transport of the skin-side sheet; and
   a step of joining an intermediate sheet and the skin-side sheet,
      the intermediate sheet having a skin-side surface that is flat compared with the protruded and recessed shape of the first region,
      joining being performed in which one part of the intermediate sheet is joined to the second region of the skin-side sheet,
      subsequently, joining being performed in which another part of the intermediate sheet is joined to the first region of the skin-side sheet.

According to the above-described method for manufacturing an absorbent article, the second region are not shaped into protruded and recessed shape, making it easier for the intermediate sheet to be joined without any wrinkles to the second region while the second region being transported in a tight state, compared with the first region. In conjunction therewith, it becomes easier for the intermediate sheet to be joined without any wrinkles to the first region while the first region being transported in a tight state.

In such a method for manufacturing an absorbent article,
a direction-of-transport length of a joining region where the second region and the intermediate sheet are joined is made longer than a direction-of-transport length of a joining region where the first region and the intermediate sheet are joined.

According to the above-described method for manufacturing an absorbent article, in the direction of transport, elongated is the joining region where the intermediate sheet and the second region, which is easily joined without any wrinkles, are joined. In conjunction therewith, the joining region where the first region and the intermediate sheet are joined becomes more likely to be joined without any wrinkles.

In such a method for manufacturing an absorbent article,
a non-joining region to which the intermediate sheet is not joined is formed in a portion of the first region located upstream in the direction of transport with respect to a joining region where the first region and the intermediate sheet are joined, and
a direction-of-transport length of the joining region where the first region and the intermediate sheet are joined is made shorter than a direction-of-transport length of the non-joining region.

According to the above-described method for manufacturing an absorbent article, the joining region where the intermediate sheet and the first region having a risk of the generation of a wrinkle are joined becomes short in the direction of transport, making it possible to reduce the generation of a wrinkle in the absorbent article.

In such a method for manufacturing an absorbent article,
a non-joining region to which the intermediate sheet is not joined is formed in a portion of the first region located upstream in the direction of transport with respect to a joining region where the first region and the intermediate sheet are joined, and
a direction-of-transport length of the joining region where the second region and the intermediate sheet are joined is made longer than a direction-of-transport length of the non-joining region.

According to the above-described method for manufacturing an absorbent article, in the direction of transport, elongated is the joining region where the intermediate sheet and the second region, which is easily joined without any wrinkles, are joined. In conjunction therewith, the joining region where the first region and the intermediate sheet are joined becomes more likely to be joined without any wrinkles.

In such a method for manufacturing an absorbent article,
the method further comprises a step of folding the absorbent article at a position of the absorbent article that overlaps in the direction of transport with a joining region where the second region and the intermediate sheet are joined.

According to the above-described method for manufacturing an absorbent article, it is possible to suppress the collapse of the protruded and recessed shape in the first region due to the folding of the absorbent article.

An absorbent article having a longitudinal direction and a lateral direction,
the absorbent article comprising:
   an absorbent body;
   a skin-side sheet located on a skin side with respect to the absorbent body; and
   an intermediate sheet located between the absorbent body and the skin-side sheet,
the skin-side sheet having a first region and a second region,
   the first region being a region that forms a protruded and recessed shape,
   the second region being a region that is adjacent to the first region on a front side in the longitudinal direction,
the first region having a larger sparseness difference of fibers in a plane direction of the skin-side sheet than the second region has,
a skin-side surface of the intermediate sheet being flat compared with the protruded and recessed shape of the first region,
the skin-side sheet having a second joining region and a first joining region,
   the second joining region being a region where one part of the intermediate sheet is joined to the second region,
   the first joining region being a region where another part of the intermediate sheet is joined to the first region.

According to the above-described absorbent article, the intermediate sheet can be first joined without any wrinkles to the second region which is likely to be transported in a tight state. In conjunction therewith, the intermediate sheet can be joined without any wrinkles to the first region in a tight state. In the second region having a small sparseness difference of fibers, it is possible to rapidly permeate urine throughout the entire region. In the first region, it is possible to take soft feces into the inside of the skin-side sheet from portions where fibers are sparse.

In such an absorbent article,
a non-skin-side surface of the first region forms a protruded and recessed shape, and
a non-skin-side surface of the second region is flat compared with the non-skin-side surface of the first region.

According to the above-described absorbent article, a space is provided on the non-skin side of the first region, making it possible to take soft feces into the space. The flat surfaces of the second region and the intermediate sheet are easily joined to each other without any wrinkles.

In such an absorbent article,
in a state where the absorbent article is unfolded and stretched,
a longitudinal length of the second joining region is longer than a longitudinal length of the first joining region.

According to the above-described absorbent article, the second joining region, which is easily joined without any wrinkles, becomes long in the longitudinal direction, and in conjunction therewith, also making it easier to join the first joining region without any wrinkles. The second joining region suitable for urination can be disposed long in the longitudinal direction, making it possible to suppress urine leakage.

In such an absorbent article,
the first region has a non-joining region that is adjacent to the first joining region on a back side in the longitudinal direction and to which the intermediate sheet is not joined, and
in a state where the absorbent article is unfolded and stretched,
   a longitudinal length of the first joining region is shorter than a longitudinal length of the non-joining region.

According to the above-described absorbent article, the first joining region, where there is a risk of the generation of wrinkles, becomes short in the longitudinal direction, making it possible to reduce the generation of wrinkles in the absorbent article. The non-joining region in which soft feces is easily taken into the inside of the skin-side sheet becomes long in the longitudinal direction, making it possible to reduce the amount of soft feces that remains on the skin-side sheet.

In such an absorbent article,
the first region has a non-joining region that is adjacent to the first joining region on a back side in the longitudinal direction and to which the intermediate sheet is not joined, and
in a state where the absorbent article is unfolded and stretched,
   a longitudinal length of the second joining region is longer than a longitudinal length of the non-joining region.

According to the above-described absorbent article, the second joining region, which is easily joined without any wrinkles, becomes long in the longitudinal direction, and in conjunction therewith, also making it easier to join the first joining region without any wrinkles. The second joining region suitable for urination can be disposed long in the longitudinal direction, making it possible to suppress urine leakage.

In such an absorbent article,
in a state where the absorbent article is unfolded and stretched,
a longitudinal center of the absorbent article overlaps the second joining region in the longitudinal direction.

According to the above-described absorbent article, the first region suitable for defecation can be disposed on the back side in the longitudinal direction.

In such an absorbent article,
in the first region, a plurality of protruding portions that protrude toward the skin side are arranged side-by-side in the lateral direction.

According to the above-described absorbent article, wrinkles that extend in the longitudinal direction can be divided by the protruding portions, making it possible to reduce the generation of wrinkles in the absorbent article. The protruding portions makes it possible to suppress the rate of soft feces that flows toward the back side in the longitudinal direction.

In such an absorbent article,
in a state where the absorbent article is unfolded and stretched,
a lateral length of the intermediate sheet is longer than half of a lateral length of the first region.

According to the above-described absorbent article, in conjunction with the second joining region which is easily joined without any wrinkles, the first joining region is more likely to be joined without any wrinkles across a broad range in the lateral direction. It is possible to rapidly transmit urine in the second joining region that is broad in the lateral direction.

In such an absorbent article,
in a state where the absorbent article is unfolded and stretched,
a lateral length of the intermediate sheet is longer than a lateral length of the first region.

According to the above-described absorbent article, in conjunction with the second joining region which is easily joined without any wrinkles, the first joining region is more likely to be joined without any wrinkles across a broad range in the lateral direction. It is possible to rapidly transmit urine in the second joining region that is broad in the lateral direction.

In such an absorbent article,
the absorbent article further comprises a pair of leak-proof wall portions that rise up toward the skin side, in two lateral side portions of the absorbent body, and
in a state where the absorbent article is unfolded and stretched,
   a lateral position of rising-up of each of the leak-proof wall portions is positioned outside in the lateral direction with respect to the first region.

According to the above-described absorbent article, the leak-proof wall portions stably rise up, making it possible for the leak-proof wall portions to dam soft feces.

### Embodiments

Embodiments will be described using tape-type disposable diapers for infants as examples of an absorbent article according to the present invention. However, the absorbent article is not limited thereto, and the present invention is also applicable to, for example, pull-on or pad-type disposable diapers, tape-type disposable diapers for adults, and the like.

### First Embodiment

### Basic configuration of tape-type disposable diaper 1

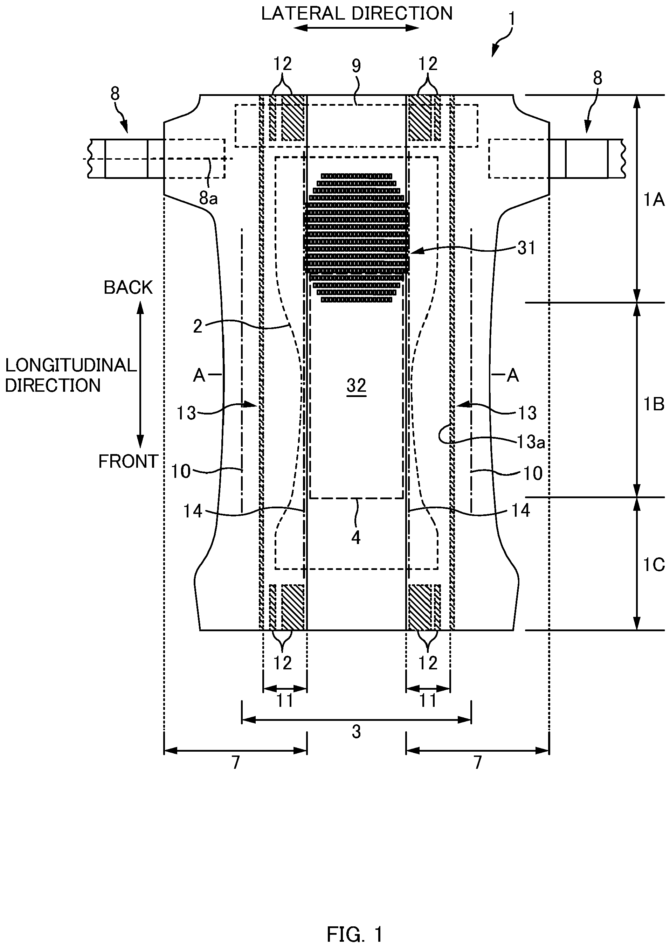

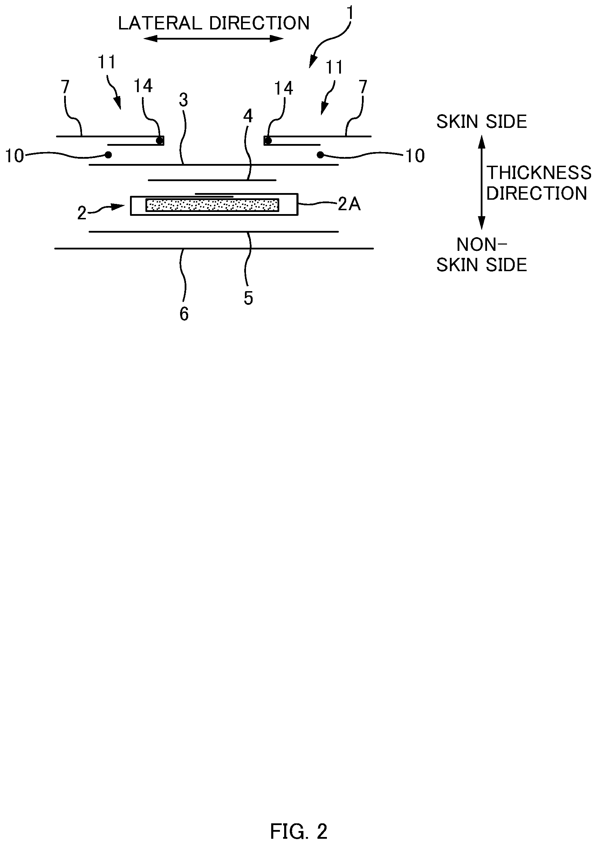

FIG. 1 is a plan view of a tape-type disposable diaper 1 (hereinafter, also referred to as a "diaper") in an unfolded and stretched state. FIG. 2 is a cross-sectional view taken along a line A-A shown in FIG. 1. The state of the diaper 1 being stretched refers to a state where the diaper 1 is stretched to an extent that wrinkles in the diaper 1 are substantially no longer visible and a state where the diaper 1 is stretched until the dimensions of constituent members thereof (e.g., the later- described side sheet 7) matches or close to the dimensions of such members on their own.

The diaper 1 has a longitudinal direction and a lateral direction that are orthogonal to each other and has a back waist region 1A, a crotch region 1B, and a front waist region 1C side by side in the longitudinal direction. As shown in FIG. 2, a direction in which constituent members of the diaper 1 are overlaid on each other will be referred to as a thickness direction. In the thickness direction, a side that comes into contact with wearer will be referred to as a skin side, and a side opposite to the skin side will be referred to as a non-skin side.

As shown in FIG. 2, the diaper 1 includes: an absorbent body 2 that absorbs excrement; a liquid-permeable skin-side sheet 3 located on the skin side with respect to the absorbent body 2; a liquid-permeable intermediate sheet 4 located between the absorbent body 2 and the skin-side sheet 3; a liquid-impermeable leak-proof sheet 5 located on the non-skin side with respect to the absorbent body 2; an exterior sheet 6 located on the non-skin side with respect to the leak-proof sheet 5; and a pair of side sheets 7 provided on two lateral side portions of the skin-side sheet 3. In addition, a pair of fastening tapes 8 extend from the back waist region 1A toward two lateral outer sides.

The absorbent body 2 can be exemplified by an absorbent body obtained by shaping a liquid-absorbent fiber such as a pulp fiber containing a superabsorbent polymer (so-called SAP) into a predetermined shape, an SAP sheet having an SAP layer attached to a hydrophilic sheet, and the like. The surface of the absorbent body 2 may be formed of a core-wrapping sheet 2A such as a liquid-permeable tissue paper or nonwoven fabric.

In the back waist region 1A, a waist stretchable member 9 that stretches and contracts in the lateral direction (e.g., a stretchable film) is disposed, and the diaper 1 fits the waist of wearer. In addition, in two lateral side portions of the diaper 1, leg stretchable members 10 that stretch and contract in the longitudinal direction (e.g., elastic strings) are disposed, and the diaper 1 fits the leg of wearer.

In addition, the diaper 1 has a pair of leak-proof wall portions 11 that rise up toward the skin side, in two lateral side portions of the absorbent body 2. The leak-proof wall portion 11 is surrounded by end joining portions 12 and a longitudinal joining portion 13, forming a pocket shape. The end joining portions 12 are portions in which two lengthwise ends of the side sheet 7 are joined to the skin-side sheet 3, and the longitudinal joining portion 13 are portions in which the side sheet 7 is joined to the skin-side sheet 3 laterally outside the end joining portions 12, through the entirety from the longitudinal one end to the longitudinal other end. In addition, at the leading end (the lateral inner end) of each leak-proof wall portion 11, a leak-proof-wall-portion stretchable member 14 that stretches and contracts in the longitudinal direction (e.g., an elastic string) is disposed. Therefore, the leak-proof wall portion 11 is capable of rising up toward the skin side from a rising-up point which is a lateral inner end 13a of the longitudinal joining portion 13.

### Surface shape of skin-side sheet 3

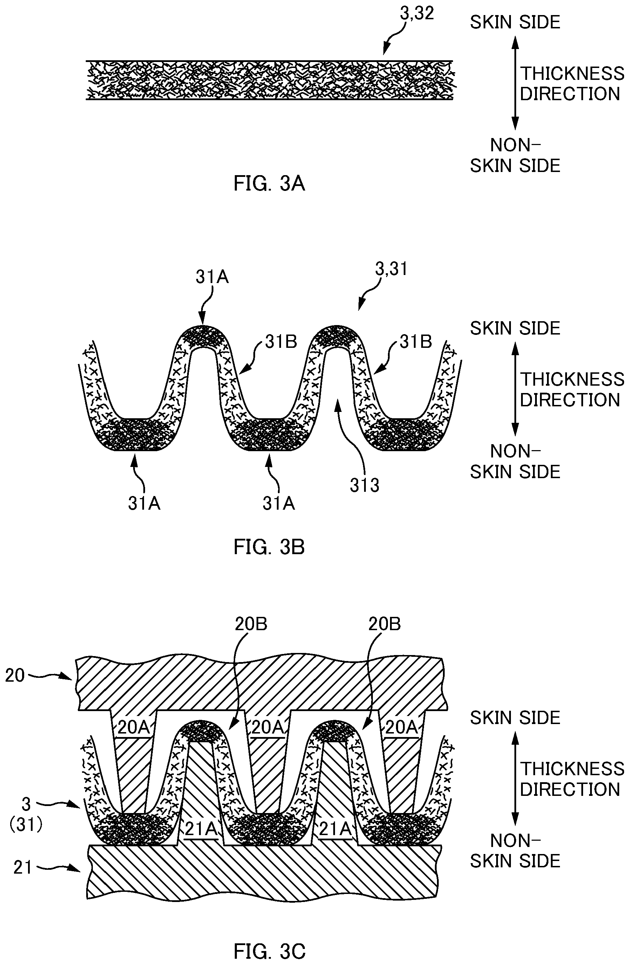

FIG. 3A is a cross-sectional view of a second surface region 32 of the skin-side sheet 3, FIG. 3B is a cross-sectional view of a first surface region 31 of the skin-side sheet 3, and FIG. 3C is a a diagram illustrating how to process the skin-side sheet 3 (first surface region 31).

The skin-side sheet 3 has the first surface region 31 and the second surface region 32; the first surface region 31 is a region in which the skin-side sheet 3 has a first surface shape, and the second surface region 32 is a region in which the skin-side sheet has a second surface shape that is different from the first surface shape. In the present embodiment, in the first surface region 31 (FIG. 3B), the skin-side surface and the non-skin-side surface of the skin-side sheet 3 have protruded and recessed shapes. On the other hand, in the second surface region 32 (FIG. 3A), the skin-side surface and the non-skin-side surface of the skin-side sheet 3 have flat shapes compared with the protruded and recessed shapes of the first surface region 31. The first surface region 31 is formed by processing (shaping into protrusions and recesses) the skin-side sheet 3 that was flat like the second surface region 32.

The processing (shaping into protrusions and recesses) the first surface region 31 can be exemplified by a method in which the skin-side sheet 3 is passed between a pair of shaping rolls 20 and 21 as shown in FIG. 3C. It should be noted that FIG. 3C is a enlarged view of parts of the shaping rolls 20 and 21. The pair of shaping rolls 20 and 21 respectively have protruding portions 20A and 21A on their own outer circumferential surfaces. While the pair of shaping rolls 20 and 21 are rotating, the protruding portions 21A of one shaping roll 21 enter recessed portions 20B located between the protruding portions 20A of the other shaping roll 20. The skin-side sheet 3 passes between these protruding portions 20A and 21A that engage each other.

As a result, in the first surface region 31, the skin-side surface and the non-skin-side surface of the skin-side sheet 3 have protruded and recessed shapes. In addition, portions that are of the skin-side sheet 3 and that are located between portions that come into contact with the protruding portions 20A and 21A of the shaping rolls are elongated in the thickness direction. Accordingly, as shown in FIG. 3B, in side wall portions 31B of the protrusions and recesses in the first surface region 31, fibers are sparse, and, in apex portions 31A of the protrusions and recesses, fibers are dense. That is, the first surface region 31 in which protrusions and recesses are shaped has a large sparseness difference of fibers in the plane direction of the skin-side sheet 3, compared with the second surface region 32 in which protrusions and recesses are not shaped.

### Absorption property of diaper 1

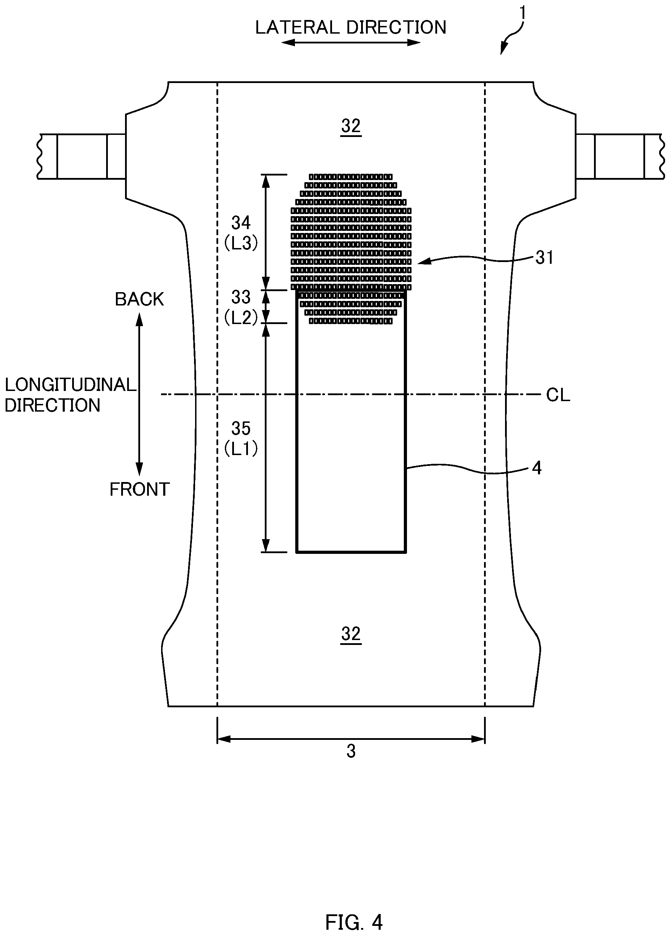

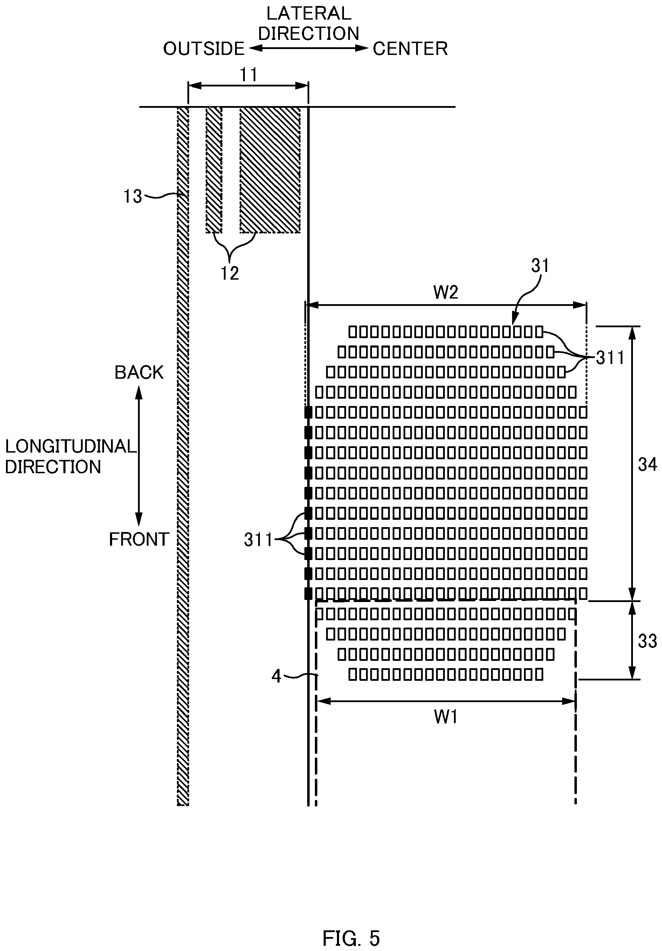

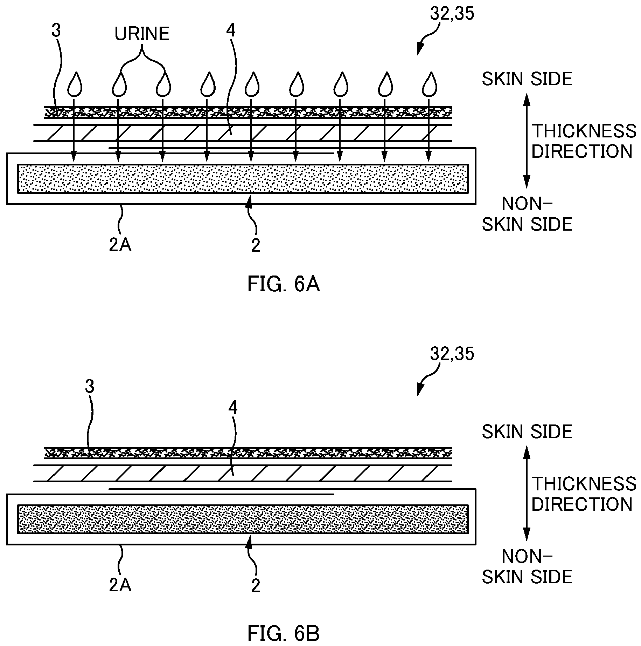

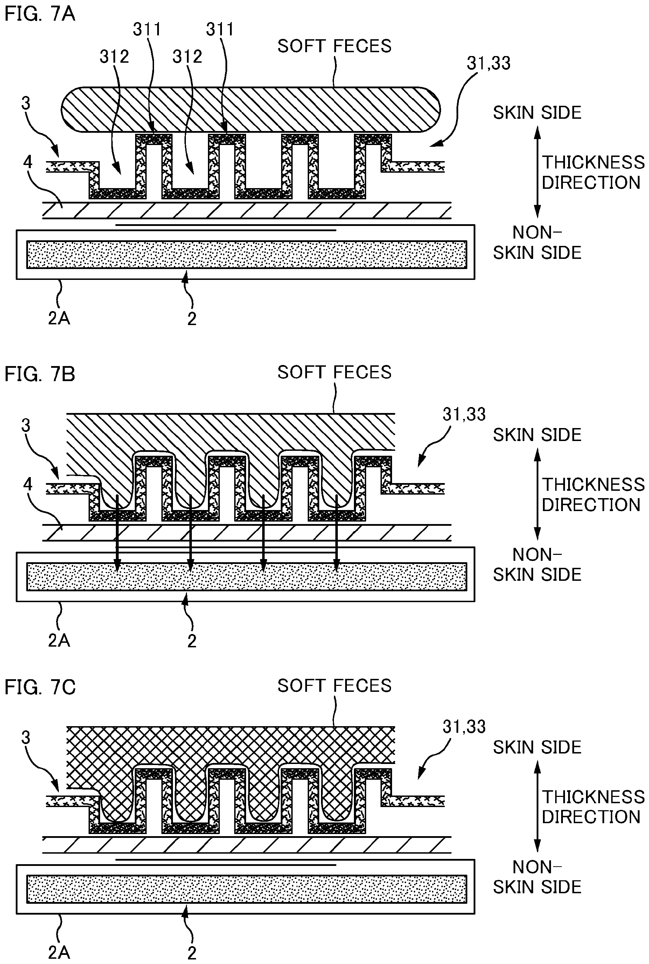

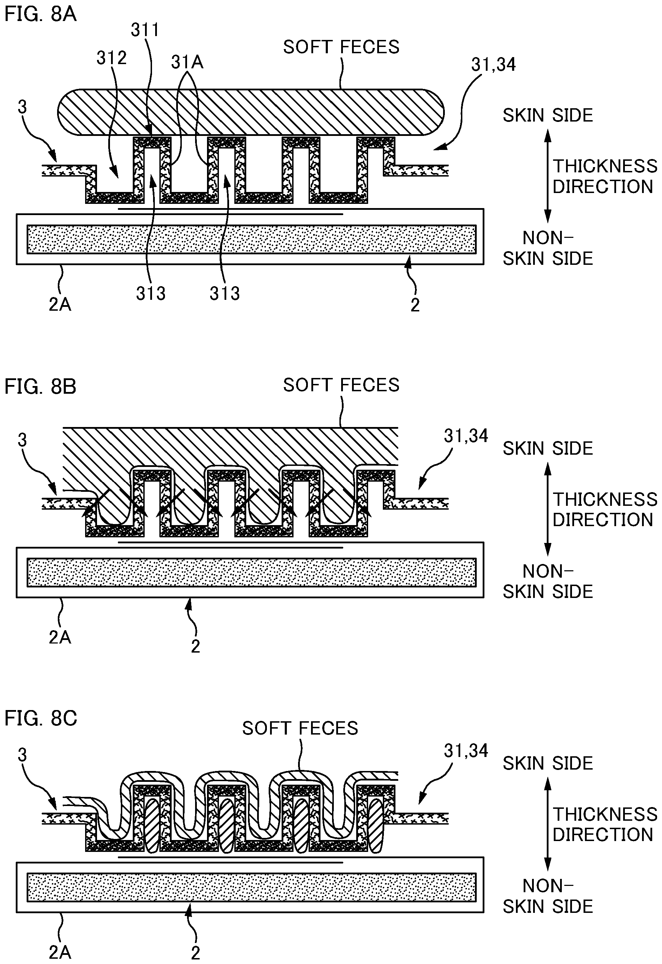

FIG. 4 is a view showing the arrangement of the first surface region 31 and the intermediate sheet 4. FIG. 5 is a view showing the arrangement of the first surface region 31 and the leak-proof wall portion 11. FIGS. 6A and 6B are explanatory diagrams illustrating how to absorb urine. FIGS. 7A to 7C and 8A to 8C are explanatory diagrams illustrating how to absorb soft feces.

As shown in FIG. 4, the skin-side sheet 3 is disposed across a broad range in the longitudinal direction and in the lateral direction of the diaper 1. The intermediate sheet 4 is smaller than the skin-side sheet 3 and is disposed in the central part of the diaper 1 in the longitudinal direction and in the lateral direction. Specifically, the intermediate sheet 4 is disposed straddling a longitudinal center CL of the diaper 1 and close to the front side in the longitudinal direction.

In addition, as described above, the skin-side sheet 3 has the first surface region 31 in which protrusions and recesses are shaped and the second surface region 32 in which protrusions and recesses are not shaped. As shown in FIG. 4, the first surface region 31 is located on the back side with respect to the longitudinal center CL of the diaper 1.

Therefore, the first surface region 31 has a first arrangement region 33 (the arrangement region of the present invention) and a first non-arrangement region 34 (the non-arrangement region of the present invention); the first arrangement region 33 is a region that overlaps the intermediate sheet 4 from the skin side and the first non-arrangement region 34 is a region that does not overlap the intermediate sheet 4. In addition, in a state where the diaper 1 is unfolded and stretched (FIG. 4), the first arrangement region 33 is located on the back side with respect to the longitudinal center CL of the diaper 1, and the first non-arrangement region 34 is located on the back side in the longitudinal direction with respect to the first arrangement region 33.

The feces of an infant (particularly, an infant whose monthly age is relatively small), who is a target wearer of the diaper 1 of the present embodiment, is soft feces. Therefore, the inventors of the present application placed artificial feces that simulated soft feces on the first arrangement region 33 and the first non-arrangement region 34 respectively, and observed them. In addition, after a predetermined period of time had elapsed, artificial skins were placed on the regions where the artificial feces had been placed, weights were placed on the artificial skins (placed were weights each of which was approximately as heavy as the body pressure of an infant whose wears the diaper). Then the amounts of the artificial feces attached to the artificial skins were observed.

As a result, the viscosity of the artificial feces that remained on the first arrangement region 33 was higher than the viscosity of the artificial feces that remained on the first non-arrangement region 34. In addition, the amount (amount per certain area) of the artificial feces that remained on the first arrangement region 33 was larger than the amount (amount per certain area) of the artificial feces that remained on the first non-arrangement region 34. Accordingly, the amount of the feces attached to the artificial skins placed on the first arrangement region 33 was larger than the amount of the feces attached to the artificial skins placed on the first non-arrangement region 34.

The theory of the above-described phenomenon is considered as follows. In the first arrangement region 33, as shown in FIGS. 7A to 7C, soft feces first enters recessed portions 312 located between protruding portions 311 that protrude on the skin side of the skin-side sheet 3. Then, moisture in the soft feces that has entered the recessed portions 312 rapidly transfers to the intermediate sheet 4 that is in contact with the recessed portions 312, increasing the viscosity of the soft feces. In addition, the permeation rate of the moisture in the soft feces is faster than the rate at which the entire soft feces is taken into the inside of the skin-side sheet 3. Accordingly, the viscosity of the soft feces increases before the entire soft feces is taken into the inside of the skin-side sheet 3. The soft feces having an increased viscosity is less likely to be taken into the inside of the skin-side sheet 3. Therefore, in the first arrangement region 33, it can be considered that the viscosity of the soft feces remaining on the surface is high and that the amount of the remaining soft feces and the amount of the soft feces attached to the skin become relatively large.

On the other hand, in the first non-arrangement region 34, as shown in FIGS. 8A to 8C, the intermediate sheet 4 is not located below the skin-side sheet 3. Accordingly, there is no case where only the moisture in the soft feces that has entered the recessed portions 312 of the skin-side sheet 3 transfers first. Consequently, the entire soft feces is taken into the inside of the skin-side sheet 3. In particular, since fibers are sparse in the side wall portions 31B of the protrusions and recesses in the skin-side sheet 3, the soft feces is taken into spaces 313 that are inside the protruding portions 311, from the side wall portions 31B. Therefore, in the first non-arrangement region 34, it can be considered that the viscosity of the soft feces remaining on the surface does not increase and that the amount of the remaining soft feces and the amount of the soft feces attached to the skin become small.

Therefore, as in the diaper 1 of the present embodiment, it is preferable that the first surface region 31 in which protrusions and recesses are shaped is located on the back side with respect to the longitudinal center CL of the diaper 1 (that is, located in a defecation region), and that the first surface region 31 has the first arrangement region 33 which overlaps the intermediate sheet 4 and the first non-arrangement region 34 which does not overlap the intermediate sheet 4. Furthermore, it is preferable that the first non-arrangement region 34 is located on the back side with respect to the first arrangement region 33.

In such a case, firstly, moisture in soft feces excreted in the first arrangement region 33 or soft feces that has flowed into the first arrangement region 33 can be rapidly transferred to the intermediate sheet 4. Accordingly, it is possible to suppress the flow (rate) of the soft feces and to suppress the leakage of the soft feces from the back side. Thereafter, the soft feces that has transferred and flowed into the first non-arrangement region 34 on the back side, beyond the first arrangement region 33 where the highly viscous soft feces remains, can be taken into the inside of the skin-side sheet 3. Therefore, it is possible to reduce the amount of the soft feces that remains in the skin-side sheet 3, making it possible to suppress the soft feces from being attached to the skin of a wearer.

The intermediate sheet 4 can be exemplified by a sheet that is coarser (have larger pores between fibers) than the absorbent body 2 (core-wrapping sheet 2A) and has a high liquid permeation rate (absorption rate). For example, the intermediate sheet 4 can be exemplified by a sheet containing air-through nonwoven fabric, spunbond nonwoven fabric, or crosslinked cellulose fiber and the like. The liquid permeation rates (absorption rates) can be compared by a well-known method. For example, a sample having the absorbent body 2 alone and a sample having the intermediate sheet 4 disposed on the absorbent body 2 are acquired. In addition, cylinders are placed on the samples, respectively, a predetermined amount of liquid is poured into each of the cylinders. And the periods of time taken from the time when the liquid begins to be poured to the time when the liquid in each cylinder has discharged are measured, thereby comparing the liquid permeation rates. Alternatively, enlarged images of cross sections of the absorbent body 2 and the intermediate sheet 4 which are cut in the thickness direction are acquired, and in the enlarged images, the cross sections are each magnified at a magnification of approximately 20 to 100 times with an electron microscope or the like. In addition, using image analysis software, calculated are the area of pore portions per certain area in the cross section of the absorbent body 2 (core-wrapping sheet 2A) and the area of pore portions per certain area in the cross section of the intermediate sheet 4, comparing them.

In addition, the first surface region 31 of the present embodiment has protruded and recessed shapes formed by the pair of shaping rolls 20 and 21 (FIG. 3C), and the density of fibers is sparser in the side wall portions 31B of the protrusions and recesses than in the apex portions 31A of the protrusions and recesses. As described above, it is preferable that the first surface region 31 has a larger sparseness difference of fibers in the plane direction of the skin-side sheet 3 than the second surface region 32 has. In such a case, it becomes easy to take soft feces to the inside of the skin-side sheet 3 from the portions of the first surface region 31 where fibers are sparse (the side wall portions 31B of the protrusions and recesses). This makes it possible to reduce the amount of soft feces that remains on the first surface region 31 (particularly, on the first non-arrangement region 34).

Alternatively, in a case where the skin-side surface of the first surface region 31 has a protruded and recessed shape, it is possible to suppress the flow of soft feces compared with a case where the skin-side surface is flat as in, for example, the second surface region 32. In addition, due to the first surface region 31 having a protruded and recessed shape, soft feces that has entered the recessed portions 312 can be moved closer to the intermediate sheet 4, making it possible to rapidly transfer moisture in soft feces to the intermediate sheet 4, and making it possible to suppress the flow of the soft feces. Therefore, it is possible to suppress the leakage of soft feces from the back side.

The sparseness differences of fibers can be compared by a well-known method. For example, enlarged images of cross sections of the first surface region 31 and the second surface region 32 which are cut in the thickness direction are acquired, and in the enlarged images, the cross sections are each magnified at a magnification of approximately 20 to 100 times with an electron microscope or the like. In addition, in the cross section of the first surface region 31, calculating the difference between the number of fibers per certain area of the portion where the fibers are dense (apex portion 31A) and the number of fibers per certain area of the portion where the fibers are sparse (side wall portion 31B), the difference is defined as the sparseness difference of fibers in the first surface region 31. Similarly, in the cross section of the second surface region 32, calculating the difference between the number of fibers per certain area of the portion where the fibers are dense and the number of fibers per certain area of the portion where the fibers are sparse, the difference is defined as the sparseness difference of fibers in the second surface region 32. It is recommended that these sparseness differences of fibers are compared with each other. Alternatively, the sparseness differences of fibers may be compared visually from the enlarged cross-sectional images.

In addition, it is preferable that, in the first surface region 31, a space is provided between the skin-side sheet 3 and the material that is adjacent to the skin-side sheet 3 on the non-skin side (the intermediate sheet 4 or the absorbent body 2). In the present embodiment, since the non-skin-side surface of the skin-side sheet 3 forms a protruded and recessed shape, the spaces 313 are provided between the protruding portions 311 that protrude toward the skin side and the material located below. In such a case, it is possible to take a larger amount of soft feces into the spaces 313 below the skin-side sheet 3, making it possible to further reduce the amount of soft feces that remains in the skin-side sheet 3.

In addition, it is preferable that in a state where the diaper 1 is unfolded and stretched (FIG. 4), the longitudinal length L3 of the first non-arrangement region 34 is longer than the longitudinal length L2 of the first arrangement region 33 (L3 > L2). In such a case, the first non-arrangement region 34, in which soft feces is easily taken into the inside of the skin-side sheet 3, can be disposed across a broad range in the longitudinal direction. This makes it possible to further reduce the amount of soft feces that remains in the skin-side sheet 3.

In addition, it is preferable that, similar to the first surface region 31, the second surface region 32 also has a second arrangement region 35 that overlaps the intermediate sheet 4 from the skin side. Furthermore, it is preferable that in a state where the diaper 1 is unfolded and stretched, at least a part of the second arrangement region 35 is positioned on the front side in the longitudinal direction with respect to the first arrangement region 33. That is, the second arrangement region 35 is preferably positioned in an urination region.

The second surface region 32 is a region where protrusions and recesses are not shaped and the sparseness difference of fibers is small. Therefore, in the second surface region 32, there is no portion where the density of fibers is high and urine is not easily transmitted, and urine is easily transmitted across the entire region. Accordingly, in the second arrangement region 35, it is possible to rapidly transfer urine to the intermediate sheet 4 in the entire region, making it possible to suppress urine leakage. In addition, due to the intermediate sheet 4, not only is the urine absorption performance enhanced, but It is also possible to suppress backflow of urine from the absorbent body 2.

Furthermore, it is preferable that in a state where the diaper 1 is unfolded and stretched (FIG. 4), the longitudinal length (L1) of the second arrangement region 35 is longer than the sum of the longitudinal length (L2) of the first arrangement region 33 and the longitudinal length (L3) of the first non-arrangement region 34 (L1 > L2 + L3). In such a case, the second arrangement region 35 suitable for urination can be disposed across a broad range in the longitudinal direction. This makes it possible to rapidly transfer urine, which has a higher fluidity than soft feces, to the intermediate sheet 4 in the longitudinally-large second arrangement region 35, making it possible to suppress urine leakage.

In addition, in the protruding portions 311 of the protruded and recessed shape formed in the first surface region 31, the density of fibers is relatively high, and the stiffness is high. In the case of the tape-type diaper 1, portions that overlap the fastening tape 8 in the longitudinal direction are in close contact with the skin of a wearer more strongly. Therefore, it is preferable that in a state where the diaper 1 is unfolded and stretched, the first surface region 31 is positioned on the front side in the longitudinal direction with respect to longitudinal centers 8a of the fastening tapes 8 as shown in FIG. 1. In such a case, it is possible to prevent for the fastening tapes 8 to make the highly stiff portions in the first surface region 31 to be in close contact with the skin of a wearer strongly, making it possible to enhance wearing comfortability.

Similarly, in the case of the tape-type diaper 1, a portion that overlaps the waist stretchable member 9 in the longitudinal direction are in close contact with the skin of a wearer more strongly. Therefore, it is preferable that in a state where the diaper 1 is unfolded and stretched, the first surface region 31 is positioned on the front side in the longitudinal direction with respect to the waist stretchable member 9 as shown in FIG. 1. In such a case, it is possible to prevent for the waist stretchable member 9 to make the highly stiff portions in the first surface region 31 to be in close contact with the skin of a wearer strongly, making it possible to enhance wearing comfortability.

In addition, in the present embodiment, in the first surface region 31, protrusions and recesses are shaped only in the skin-side sheet 3, and protrusions and recesses are not shaped in the intermediate sheet 4. Therefore, compared with a case where protrusions and recesses are shaped in the intermediate sheet 4 as well as in the skin-side sheet 3, the shaping of protrusions and recesses does not excessively increase the density of fibers, decreasing the stiffness and making it possible to enhance wearing comfortability. However, the configuration is not limited thereto. Protrusions and recesses may be shaped in the intermediate sheet 4 as well as in the skin-side sheet 3 in the first surface region 31.

In addition, it is preferable that in the first surface region 31, the plurality of protruding portions 311 that protrude toward the skin side are arranged side-by-side at intervals in the lateral direction as shown in FIG. 5. When the portions that protrude toward the skin side in the first surface region 31 have a predetermined lateral length as described above, it is possible to suppress the rate of soft feces that flows toward the back side in the longitudinal direction. Therefore, it is possible to suppress the leakage of soft feces from the back side.

Furthermore, it is preferable that the plurality of protruding portions 311 arranged in the lateral direction are arranged in the longitudinal direction. That is, it is preferable that the plurality of protruding portions 311 are arranged side-by-side at intervals in the longitudinal direction as well. When the portions that protrude toward the skin side in the first surface region 31 have a predetermined longitudinal length as described above, it is possible to suppress the rate of soft feces that flows in the lateral direction. Therefore, it is possible to suppress the leakage of soft feces from the leg circumference openings.

It should be noted that the protruded and recessed shape of the first surface region 31 is not limited to the protruded and recessed shape shown in FIG. 5. For example, a plurality of laterally-elongated protruding portions in which the plurality of protruding portions 311 arranged side-by-side in the lateral direction are integrated may be arranged side-by-side in the longitudinal direction. In addition, in FIG. 5, the lateral positions of the protruding portions 311 which are adjacent to each other in the longitudinal direction are aligned, but may not be aligned.

In addition, as shown in FIG. 5, it is preferable that in a state where the diaper 1 is unfolded and stretched, the lateral length W2 of the first non-arrangement region 34 is longer than the lateral length W1 of the first arrangement region 33 (W2 > W1). In such a case, soft feces that has spread in the lateral direction can be taken into the inside of the skin-side sheet 3 in the first non-arrangement region 34. This makes it possible to further reduce the amount of soft feces that remains in the skin-side sheet 3.

In addition, it is preferable that in a state where the diaper 1 is unfolded and stretched, at least a part of the portion of the first non-arrangement region 34 that extends outward in the lateral direction with respect to the first arrangement region 33 (in the case of FIG. 5, the black-colored protruding portions 311) is positioned on the central side in the lateral direction with respect to the leak-proof wall portion 11 as shown in FIG. 5. In such a case, in the first non-arrangement region 34 between the first arrangement region 33 and the leak-proof wall portion 11 in the lateral direction, soft feces can be guided to the inner side of the leak-proof wall portion 11 while taking the soft feces into the inside of the skin-side sheet 3. Therefore, the soft feces is easily dammed by the leak-proof wall portion 11, making it possible to suppress the leakage of the soft feces from the leg circumference opening.

It should be noted that, in FIG. 5, the first arrangement region 33 and the first non-arrangement region 34 do not overlap each other in the longitudinal direction, but the configuration is not limited thereto. While not shown, the following configuration is also acceptable: in a position that overlaps the first arrangement region 33 in the longitudinal direction, the first surface region 31 extends outward in the lateral direction with respect to the intermediate sheet 4, and the first arrangement region 33 and the first non-arrangement region 34 are positioned side-by-side in the lateral direction.

### Modified examples of skin-side sheet 3

### First modified example

The following configuration is acceptable: the first surface region 31 has a protruded and recessed shape on either one of the skin-side surface and the non-skin-side surface; and the first surface region 31 has a shape that is flat compared with the protruded and recessed shape, on the other one of the skin-side surface and the non-skin-side surface (not shown). Such a first surface region 31 is formed with, for example, a pair of shaping rolls 20 and 21 in which protruding portions are provided on the outer circumferential surface of only either one of the pair of shaping rolls 20 and 21 and in which the outer circumferential surface of the other one of the pair of shaping rolls 20 and 21 is flat.

Even in this case, the surface shape of the first surface region 31 is different from the surface shape of the second surface region 32 in which protrusions and recesses are not shaped (e.g., FIG. 3A). In addition, the sparseness difference of fibers is large in the first surface region 31 compared with the second surface region 32. Furthermore, in the first arrangement region 33 that overlaps the intermediate sheet 4 in the first surface region 31, it is possible to transfer moisture in soft feces 4 to the intermediate sheet 4 and to suppress the flow of soft feces. On the other hand, in the first non-arrangement region 34 that does not overlap the intermediate sheet 4 in the first surface region 31, it is possible to take soft feces into the inside of the skin-side sheet 3 from the portions where fibers are sparse.

Particularly, in a case where the skin-side surface has a protruded and recessed shape and the non-skin-side surface is flat in the first surface region 31, the protruded and recessed shape of the skin-side surface can suppress the flow of soft feces with. Conversely, in a case where the skin-side surface is flat and the non-skin-side surface has a protruded and recessed shape in the first surface region 31, it is possible to provide a space below the skin-side sheet 3, making it possible to take a larger amount of soft feces into the space.

### Second modified example

The first surface region 31 may have a through hole that penetrates the skin-side sheet 3 in the thickness direction (not shown). Such a first surface region 31 is formed by, for example, penetrating the first surface region 31 with a needle-like projection.

Even in this case, the surface shape of the first surface region 31 is different from the surface shape of the second surface region 32 having no through hole (e.g., FIG. 3A). In addition, there are almost no fibers in the through hole in the first surface region 31, and therefore the sparseness difference of fibers is large between the portion of the through hole and a portion excluding the portion of the through hole. Consequently, the sparseness difference of fibers in the first surface region 31 is larger than that in the second surface region 32. Furthermore, in the first arrangement region 33 that overlaps the intermediate sheet 4 in the first surface region 31, soft feces can come into contact with the intermediate sheet 4 in the through hole, making it possible to transfer moisture in soft feces to the intermediate sheet 4 and making it possible to suppress the flow of soft feces. On the other hand, in the first non-arrangement region 34 that does not overlap the intermediate sheet 4 in the first surface region 31, it is possible to take soft feces into the inside of the diaper 1 from the through hole.

### Third modified example

Hitherto, the second surface region 32 has a flat shape, but the configuration is not limited thereto. The skin-side surface of the material that forms the skin-side sheet 3 may be made of a nonwoven fabric having a protruded and recessed shape (e.g., a nonwoven fabric having a protruded and recessed shape formed by blowing fluid to a fiber web). In this case, the skin-side surface of the second surface region 32 also has the protruded and recessed shape. Even in this case, a processing such as the shaping of protrusions and recesses or the formation of a through hole is performed to a nonwoven fabric having a protruded and recessed shape on the skin-side surface, forming the first surface region 31, and thereby the same effect as described above can be obtained. In addition, after the same processing (the shaping of protrusions and recesses or the formation of a through hole) is performed to the first surface region 31 and the second surface region 32, additional processing may be performed only to the first surface region 31. Even in this case, the same effect as described above can be obtained.

### Fourth modified example

In the above embodiment, the intermediate sheet 4 overlaps the first surface region 31 and the second surface region 32, but the configuration is not limited thereto. For example, the following configuration is also acceptable: the first surface region 31 extends in the front side in the longitudinal direction, and the intermediate sheet 4 overlaps only the first surface region 31 and not overlap the second surface region 32. Also in this case, it is sufficient that a part of the first arrangement region 33 that overlaps the intermediate sheet 4 in the first surface region 31 is positioned on the back side with respect to the longitudinal center CL of the diaper 1, and that at least a part of the first non-arrangement region 34 that does not overlap the intermediate sheet 4 is positioned on the back side in the longitudinal direction with respect to the first arrangement region 33.

### Fifth modified example

The second surface region 32 may have a larger sparseness difference of fibers in the plane direction of the skin-side sheet 3 than the first surface region 31. For example, it is acceptable that the second surface region 32 is processed (is shaped into protrusions and recesses, or undergoes the formation of through holes) but the first surface region 31 is not processed. Even in this case, transferring moisture in the soft feces to the intermediate sheet 4 makes it possible to suppress the flow of soft feces in the first arrangement region 33, while the flow of soft feces being suppressed by the protrusions and recesses in the second surface region 32 or the like. In addition, in the first non-arrangement region 34, since moisture in soft feces does not transfer first, it is possible to take the entire soft feces into the inside of the skin-side sheet 3.

Hitherto, the above embodiment has been described in order to facilitate the understanding of the present invention, not to interpret the present invention in a limited manner. The present disclosure may variously be changed or altered without departing from its gist and encompass equivalents thereof.

For example, the followings are not limited to the above-mentioned examples: the arrangement positions of the first surface region 31 and the second surface region 32, the relationships between the lengths of the first arrangement region 33, the first non-arrangement region 34, and the second arrangement region 35 in the longitudinal direction and in the lateral direction, the positional relationship between the first non-arrangement region 34 and the leak-proof wall portion 11, and the like.

### Second Embodiment

### Basic configuration of tape-type disposable diaper 100

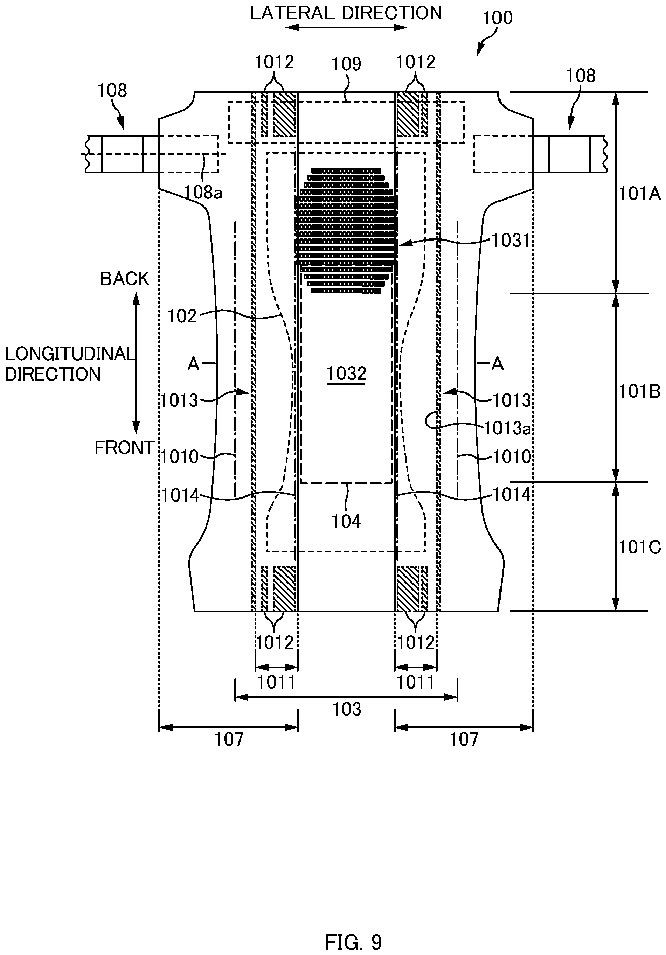

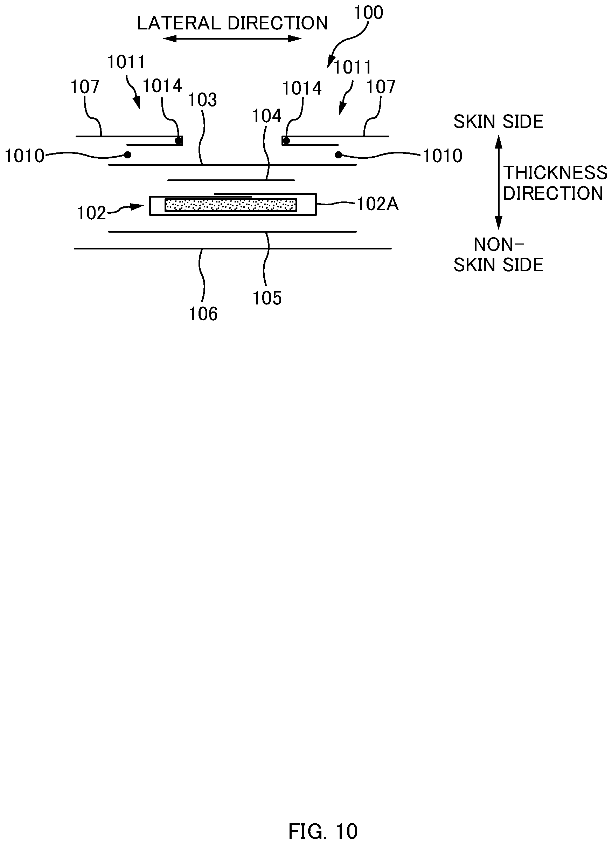

FIG. 9 is a plan view of a tape-type disposable diaper 100 (hereinafter, also referred to as a "diaper") in an unfolded and stretched state. FIG. 10 is a cross-sectional view taken along a line A-A shown in FIG. 9. The state of the diaper 100 being stretched refers to a state where the diaper 100 is stretched to an extent that wrinkles in the diaper 100 are substantially no longer visible and a state where the diaper 100 is stretched until the dimension of constituent member thereof (e.g., the later- described side sheet 107) matches or close to the dimensions of such members on their own.

The diaper 100 has a longitudinal direction and a lateral direction that are orthogonal to each other and has a back waist region 101A, a crotch region 101B, and a front waist region 101C side by side in the longitudinal direction. As shown in FIG. 2, a direction in which constituent members of the diaper 100 are overlaid on each other will be referred to as a thickness direction. In the thickness direction, a side that comes into contact with wearer will be referred to as a skin side, and a side opposite to the skin side will be referred to as a non-skin side.

As shown in FIG. 2, the diaper 101 includes: an absorbent body 102 that absorbs excrement; a liquid-permeable skin-side sheet 103 located on the skin side with respect to the absorbent body 102; a liquid-permeable intermediate sheet 104 located between the absorbent body 102 and the skin-side sheet 103; a liquid-impermeable leak-proof sheet 105 located on the non-skin side with respect to the absorbent body 102; an exterior sheet 106 located on the non-skin side with respect to the leak-proof sheet 105; and a pair of side sheets 107 provided on two lateral side portions of the skin-side sheet 103. In addition, a pair of fastening tapes 108 extends from the back waist region 1A toward two lateral outer sides.

The absorbent body 102 can be exemplified by an absorbent body obtained by shaping a liquid-absorbent fiber such as a pulp fiber containing a superabsorbent polymer (so-called SAP) into a predetermined shape, an SAP sheet having an SAP layer attached to a hydrophilic sheet, and the like. The surface of the absorbent body 102 may be formed of a core-wrapping sheet 102A such as a liquid-permeable tissue paper or nonwoven fabric.

In the back waist region 101A, a waist stretchable member 109 that stretches and contracts in the lateral direction (e.g., a stretchable film) is disposed, and the diaper 101 fits the waist of wearer. In addition, in two lateral side portions of the diaper 101, leg stretchable members 1010 that stretch and contract in the longitudinal direction (e.g., elastic strings) are disposed, and the diaper 101 fits the leg of wearer.

In addition, the diaper 101 has a pair of leak-proof wall portions 1011 that rise up toward the skin side in two lateral side portions of the absorbent body 102. The leak-proof wall portion 1011 is surrounded by end joining portions 1012 and a longitudinal joining portion 1013, forming a pocket shape. The end joining portions 1012 are portions in which two lengthwise ends of the side sheet 107 are joined to the skin-side sheet 103, and the longitudinal joining portion 1013 are portions in which the side sheet 107 is joined to the skin-side sheet 103 laterally outside the end joining portions 1012, through the entirety from the longitudinal one end to the longitudinal other end. In addition, at the leading end (the lateral inner end) of each leak-proof wall portion 1011, a leak-proof-wall-portion stretchable member 1014 that stretches and contracts in the longitudinal direction (e.g., an elastic string) is disposed. Therefore, the leak-proof wall portion 1011 is capable of rising up toward the skin side from a rising-up point which is a lateral inner end 1013a of the longitudinal joining portion 1013.

### Method for manufacturing diaper 100

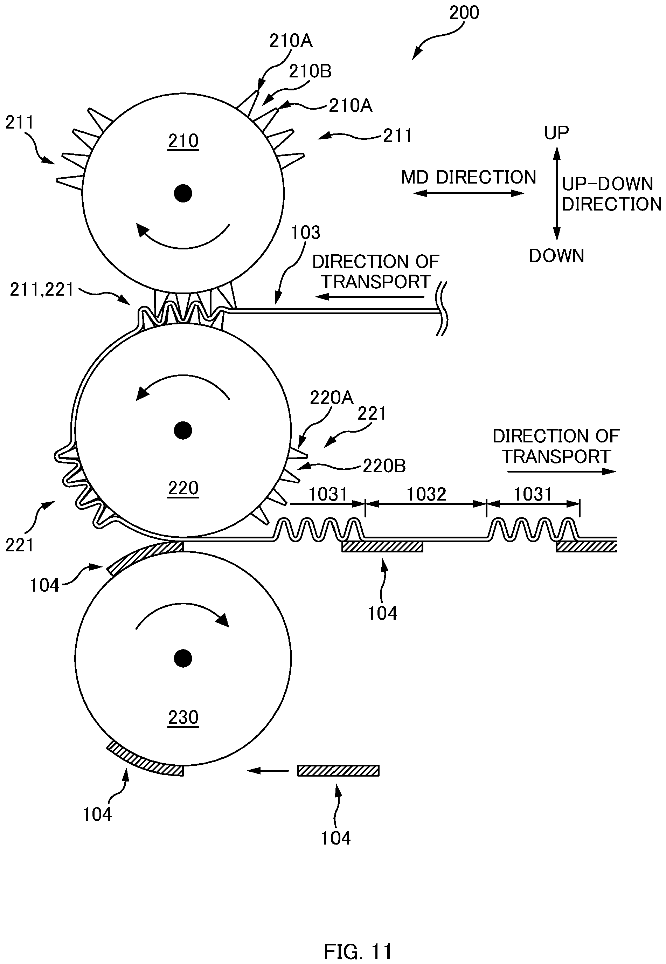

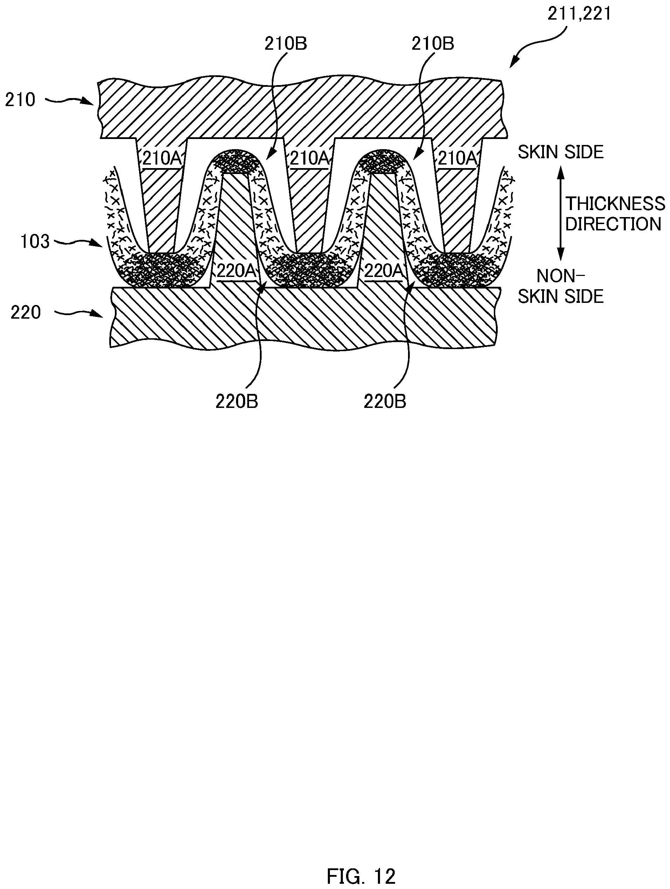

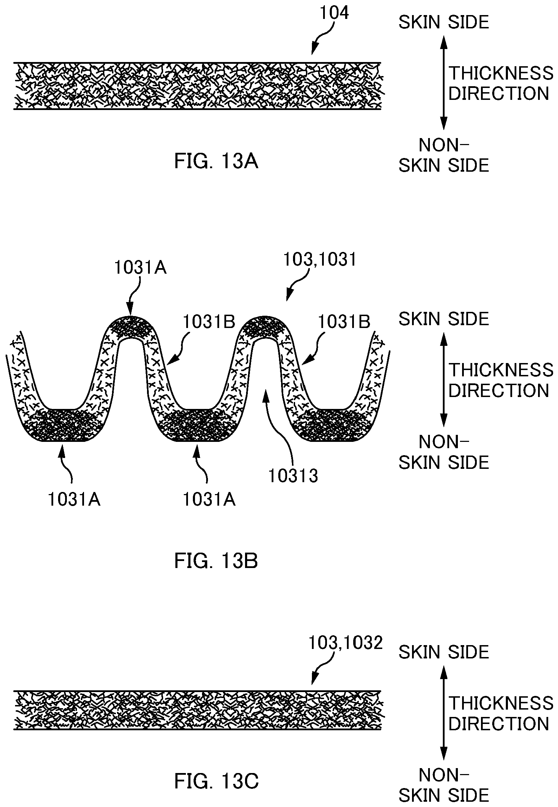

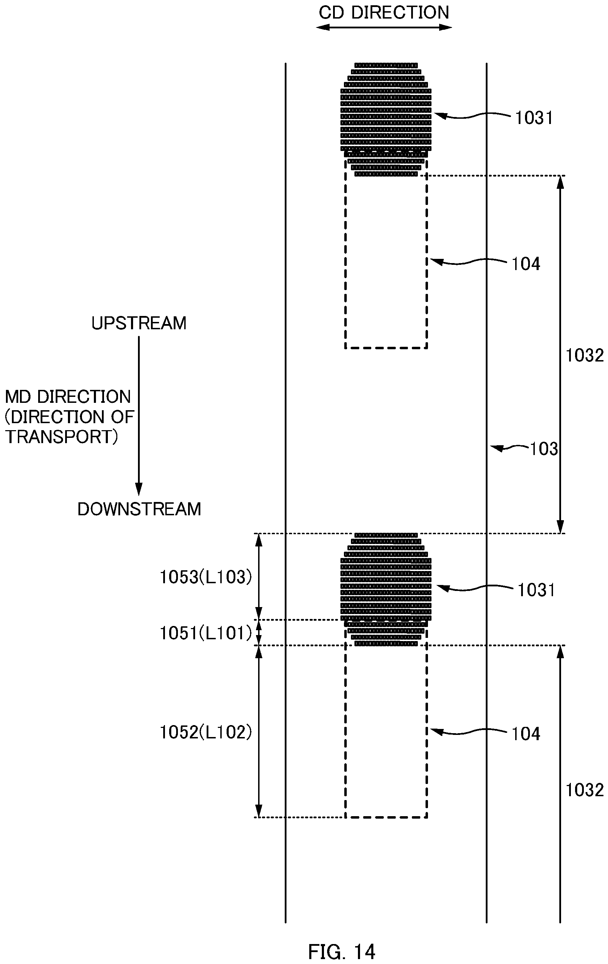

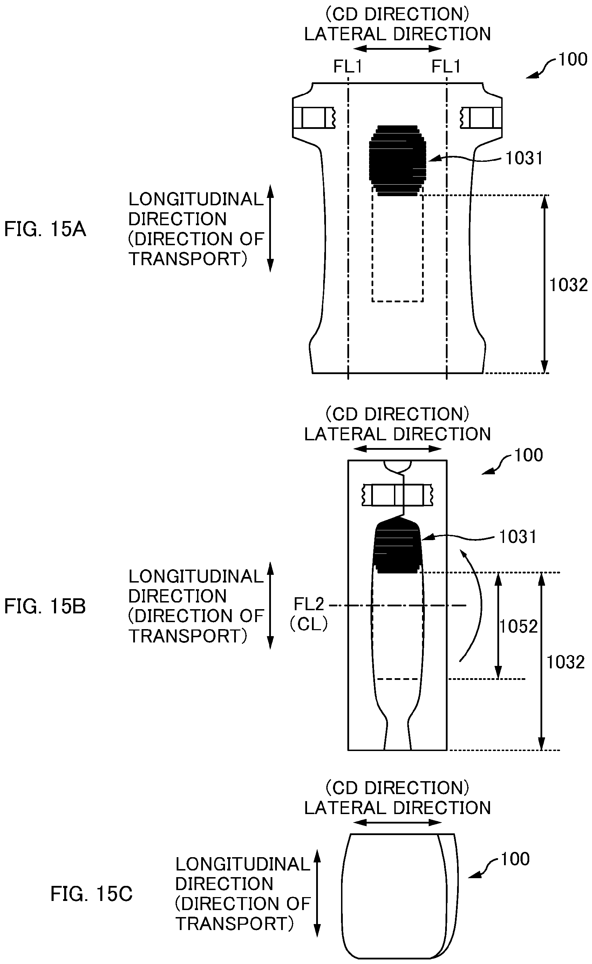

FIG. 11 is a view schematically showing a part of a manufacturing apparatus 200 of the diaper 100. FIG. 12 is an enlarged view of protrusion-and-recess-shaping portions 211 and 221. FIG. 13A is a cross-sectional view of the intermediate sheet 104, FIG. 13B is a cross-sectional view of a first region 1031 of the skin-side sheet 103, and FIG. 13C is a cross-sectional view of a second region 1032 of the skin-side sheet 103. FIG. 14 is a diagram illustrating a state where the intermediate sheets 104 are joined to the skin-side sheets 103 (continuous body). FIGS. 15A to 15C are explanatory diagrams illustrating a folding step of the diaper 100.

### Manufacturing apparatus 200 of diaper 100

The manufacturing apparatus 200 of the diaper 100 has a pair of shaping rolls 210 and 220 and a joining roll 230. One of the pair of shaping rolls will be referred to as a first shaping roll 210, and the other will be referred to as a second shaping roll 220. In the manufacturing apparatus 200 of the diaper 100, the direction of transport of materials is defined as an MD direction, a direction that is orthogonal to the MD direction and is along the plane of the material is defined as a CD direction, and a direction that is orthogonal to the MD direction and the CD direction is defined as an up-down direction.

The pair of shaping rolls 210 and 220 and the joining roll 230 are rolls that rotate in a state where the axial direction is in the CD direction. The first shaping roll 210, the second shaping roll 220, and the joining roll 230 are sequentially disposed from above. The first shaping roll 210 and the second shaping roll 220 are disposed facing each other, and the second shaping roll 220 and the joining roll 230 are disposed facing each other.

On the outer circumferential surface of each of the first shaping roll 210 and the second shaping roll 220, a plurality of protrusion-and-recess-shaping portions 211 or 221 are intermittently provided in the direction of rotation. In the protrusion-and-recess-shaping portion 211 (221), a plurality of recessed portions 210B (220B) are provided between a plurality of protruding portions 210A (220A). The protrusion-and-recess-shaping portions 211 and 221 of both rolls 210 and 220 are disposed so as to engage each other at the facing position of both rolls 210 and 220.

### Method for manufacturing diaper 100

A method for manufacturing the diaper 100 includes: a shaping step of shaping the skin-side sheet 103 into a protruded and recessed shape; a joining step of joining the intermediate sheet 104 and the skin-side sheet 103; and a folding step of folding the diaper 100 for packaging.

### (Shaping step)

In the shaping step, a continuous body of the skin-side sheet 103 is fed between the pair of shaping rolls 210 and 220 (hereinafter, the continuous body of the skin-side sheet 103 will also be simply referred to as the skin-side sheet 103). The direction in which the skin-side sheet 103 continues corresponds to the MD direction of the manufacturing apparatus 200, and the width direction of the skin-side sheet 103 corresponds to the CD direction of the manufacturing apparatus 200. As described above, the protrusion-and-recess-shaping portions 211 and 221 of both rolls 210 and 220 engage each other between the pair of shaping rolls 210 and 220. As shown in FIG. 12, the protruding portions 220A of the second shaping roll 220 enter the recessed portions 21B of the first shaping roll 210, and the protruding portions 210A of the first shaping roll 210 enter the recessed portions 22B of the second shaping roll 220. Consequently, the skin-side surface and the non-skin-side surface of the skin-side sheet 103 that passes between the pair of shaping rolls 210 and 220 have protruded and recessed shapes.

Here, in the pair of shaping rolls 210 and 220, the protrusion-and-recess-shaping portions 211 and 221 are intermittently disposed. Therefore, when the skin-side sheet 103 passes between the pair of shaping rolls 210 and 220, in a case where the protrusion-and-recess-shaping portions 211 and 221 of both rolls 210 and 220 face each other, the skin-side sheet 103 is shaped into a protruded and recessed shape. In a case where portions excluding the protrusion-and-recess-shaping portions 211 and 221 of both rolls 210 and 220 face each other, the skin-side sheet 103 is not shaped into a protruded and recessed shape.

That is, in the shaping step, the skin-side sheet 103 is passed between the pair of shaping rolls 210 and 220, thereby shaping the first region 1031 (FIG. 13B) of the skin-side sheet 103 into a protruded and recessed shape, but not shaping the second region 1032 (FIG. 13C) of the skin-side sheet 103, which is adjacent the first region 1031 in the MD direction (direction of transport), into a protruded and recessed shape. In the second region 1032 in which protrusions and recesses are not shaped, the skin-side surface and the non-skin-side surface form shapes that are flat compared with the protruded and recessed shape of the first region 1031.

It should be noted that, preferably, the pair of shaping rolls 210 and 220 are heated in advance or a preheated skin-side sheet 103 is fed between the pair of shaping rolls 210 and 220. In such a case, the protruded and recessed shape of the first region 1031 of the skin-side sheet 103 is easily maintained.

### (Joining step)

The skin-side sheet 103 having protrusions and recesses shaped in the first region 1031 is continuously transported along the outer circumferential surface of the second shaping roll 220. On the other hand, the intermediate sheet 104 is cut to a product size and then transported along the outer circumferential surface of the joining roll 230. Protrusions and recesses are not shaped in the intermediate sheet 104 with the pair of shaping rolls 210 and 220. Therefore, the skin-side surface and the non-skin-side surface of the intermediate sheet 104 (FIG. 13A) are flat compared with the protruded and recessed shape of the first region 1031 of the skin-side sheet 103. In addition, in the present embodiment, the intermediate sheet 104 is disposed in a part of the diaper 100 in the longitudinal direction as shown in FIG. 9. Therefore, on the outer circumferential surface of the joining roll 230, a plurality of the intermediate sheets 104 are intermittently transported in the direction of rotation.

In addition, when the skin-side sheet 103 and the intermediate sheets 104 pass between the second shaping roll 220 and the joining roll 230, both sheets 103 and 104 are compressed and joined to each other by both rolls 220 and 230. It should be noted that, as the joining method, it is possible to employ a well-known joining method such as an adhesive, thermal welding, or ultrasonic welding.

At this time, as shown in FIG. 11, one part of the intermediate sheet 104 is joined to the second region 1032 of the skin-side sheet 103, and then another part of the intermediate sheet 104 is joined to the first region 1031 of the skin-side sheet 103. Therefore, as shown in FIG. 14, a second joining region 1052 that is the joining region where the second region 1032 of the skin-side sheet 103 and the intermediate sheet 104 are joined is positioned downstream in the direction of transport with respect to a first joining region 1051 that is the joining region where the first region 1031 of the skin-side sheet 103 and the intermediate sheet 104 are joined.

### (Folding step)

Subsequently, the absorbent body 102, the leak-proof sheet 105, and the like are joined to the sheet where the skin-side sheet 103 and the intermediate sheet 104 are joined, manufacturing the diaper 100 in an unfolded state shown in FIG. 9. In a state where the lateral direction of the diaper 100 corresponds to the CD direction of the manufacturing apparatus 200, and where the longitudinal direction of the diaper 100 corresponds to the MD direction (direction of transport) of the manufacturing apparatus 200, the diaper 100 is folded as shown in FIGS. 15A to 15C. First, two lateral (CD-direction) side portions of the diaper 100 are folded inward in the lateral direction at folding lines FL1, and then the diaper 100 is folded in half in the longitudinal direction at a folding line FL2 in the longitudinal (direction-of-transport) center CL of the diaper 100. In this state, the diaper 100 is packaged. The method for folding the diaper for packaging shown in FIGS. 15A to 15C is an example and is not limited thereto.

### Feature portions of manufacturing method

In the case of shaping protrusions and recesses by passing the sheet between the pair of shaping rolls 210 and 220 as described above, the sheet is stretched in a plurality of directions, and a sparseness difference of fibers is caused in the sheet. For example, portions in the skin-side sheet 103 between the portions that come into contact with the protruding portions 210A and 220A of the shaping rolls 210 and 220 are stretched in the thickness direction. Therefore, as shown in FIG. 13B, in side wall portions 1031B of the protrusions and recesses in the first region 1031, fibers become sparse, and, in apex portions 1031A of the protrusions and recesses, fibers become dense. That is, in the first region 1031 in which protrusions and recesses are shaped, the sparseness difference of fibers in the plane direction of the skin-side sheet 103 becomes large, compared with the second region 1032 in which protrusions and recesses are not shaped.

In the first region 1031 in which the sparseness difference of fibers is large, a tensile force for transporting the skin-side sheet 103 is unevenly applied, making it more likely to loosen the skin-side sheet 103. When the intermediate sheet 104 is joined to the first region 1031 of the loosened skin-side sheet 103, a wrinkle is likely to be generated in the joined portion. In particular, the skin-side sheet 103 and the intermediate sheet 104 have low stiffness and are likely to loosen compared with the absorbent body 102. Therefore, in the present embodiment where the sheets 103 and 104, which are likely to be loosened, are joined to each other, a wrinkle is likely to be generated compared with a case where the absorbent body 102 is joined to the skin-side sheet 103.

Therefore, as in the manufacturing method of the present embodiment, it is preferable that one part of the intermediate sheet 104 is joined to the second region 1032 of the skin-side sheet 103, and subsequently another part of the intermediate sheet 104 is joined to the first region 1031 of the skin-side sheet 103.

In the second region 1032 of the skin-side sheet 103 where protrusions and recesses are not shaped and the sparseness difference of fibers is small, the tensile force for transporting the skin-side sheet 103 is likely to be evenly applied. Accordingly, one part of the intermediate sheet 104 is more likely to be joined to the second region 1032 which is in a tight state, without any wrinkles. In the joined portion where the second region 1032 and the intermediate sheet 104 are joined to each other without any wrinkles, stiffness increases. Therefore, the first region 1031 of the skin-side sheet 103 and another part of the intermediate sheet 104, which are positioned upstream of the joined portion, behave in conjunction with the tight state of the previously joined portion and are not easily loosened. This makes it possible to join the first region 1031 of the skin-side sheet 103 and another part of the intermediate sheet 104 in a tight state, makes it less likely to generate wrinkles in the joined portion.

In addition, the intermediate sheet 104 are not shaped into protruded and recessed shape together with the skin-side sheet 103, and the skin-side surface of the intermediate sheet 104 is flat. Similarly, the second region 1032 of the skin-side sheet 103 are not shaped into protruded and recessed shape, and the skin-side surface of the second region 1032 is flat. Accordingly, the flat surfaces of the second region 1032 and the intermediate sheet 104 are securely joined to each other without any wrinkles. In conjunction therewith, the first region 1031 and the intermediate sheet 104 are also easily joined to each other without any wrinkles.

In addition, since the intermediate sheet 104 are not shaped into protruded and recessed shape together with the skin-side sheet 103, the densities of the portions where fibers become dense due to the shaping of protrusions and recesses do not excessively increase, making it possible to decrease stiffness. Therefore, it is possible to soften the protrusion-and-recess-shaping portion (first region 1031) and to enhance the wearing comfortability of the diaper 100.

In addition, it is preferable that in the joining step, as shown in FIG. 14, the direction-of-transport length (L102) of the joining region where the second region 1032 of the skin-side sheet 103 and the intermediate sheet 104 are joined (second joining region 1052) is made longer than the direction-of-transport length (L101) of the joining region where the first region 1031 of the skin-side sheet 103 and the intermediate sheet 104 are joined (first joining region 1051) (L102 > L101). In such a case, the second joining region 1052, which is easily joined without any wrinkles while being transported in a tight state, becomes long in the direction of transport, and in conjunction thereof, also making it more easier to join the first joining region 1051 without any wrinkles.

In addition, the following configuration is acceptable: a non-joining region 1053 to which the intermediate sheet 104 is not joined is formed in a portion of the first region 1031 located upstream in the direction of transport with respect to the first joining region 1051. Even in this case, it is preferable that the direction-of-transport length (L102) of the second joining region 1052 is made longer than the direction-of-transport length (L103) of the non-joining region 1053 (L102 > L103). In such a case, the second joining region 1052, which is easily joined without any wrinkles while being transported in a tight state, becomes long in the direction of transport, and in conjunction thereof, also making it more easier to join the first joining region 1051 without any wrinkles.

In addition, to the non-joining region 1053, the intermediate sheet 104 is not joined, but the highly stiff absorbent body 102 is directly joined. Accordingly, a wrinkle is not easily generated although the non-joining region 1053 is a region where protrusions and recesses are shaped. Therefore, it is preferable that the direction-of-transport length (L101) of the first joining region 1051 is made shorter than the direction-of-transport length (L103) of the non-joining region 1053 (L101 < L103). In such a case, the first joining region 1051 where there is a risk of the generation of a wrinkle becomes short in the direction of transport, making it possible to further reduce the generation of a wrinkle in the diaper 100.

In addition, it is preferable that in the folding step, as shown in FIG. 15B, the diaper 100 is folded in the longitudinal direction at a position of the diaper 100 that overlaps the second joining region 1052 in the longitudinal direction (direction of transport) of the diaper 100. In such a case, no folding line is positioned in the first region 1031 in which protrusions and recesses are shaped, making it possible to suppress the collapse of the protruded and recessed shape. In addition, the intermediate sheet 104 is disposed in the second joining region 1052 and the second joining region 1052 is relatively highly stiff, making it possible to securely fold the diaper 100.

### Diaper 100 manufactured by method for manufacturing diaper 100

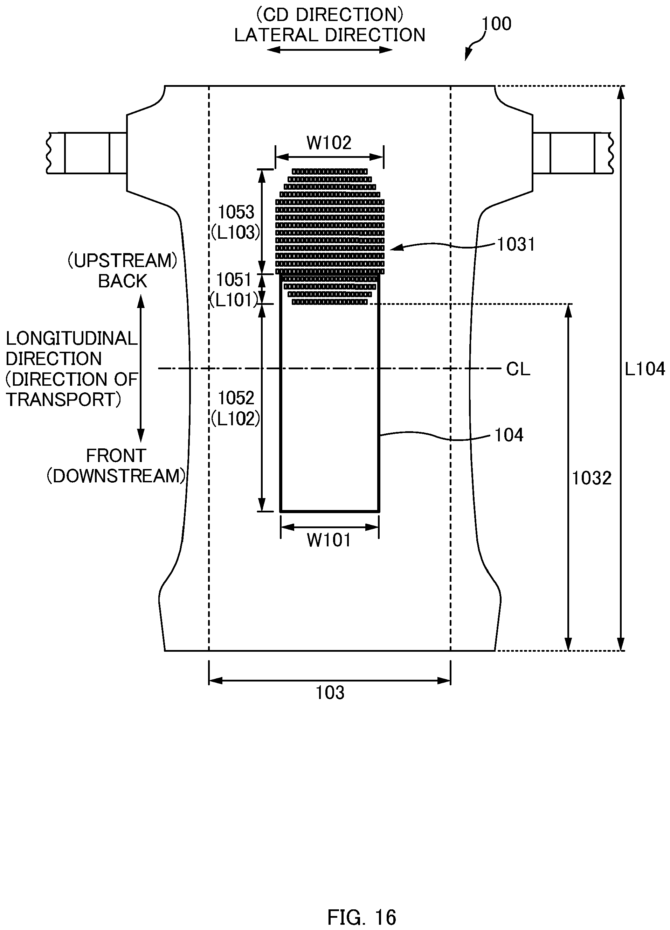

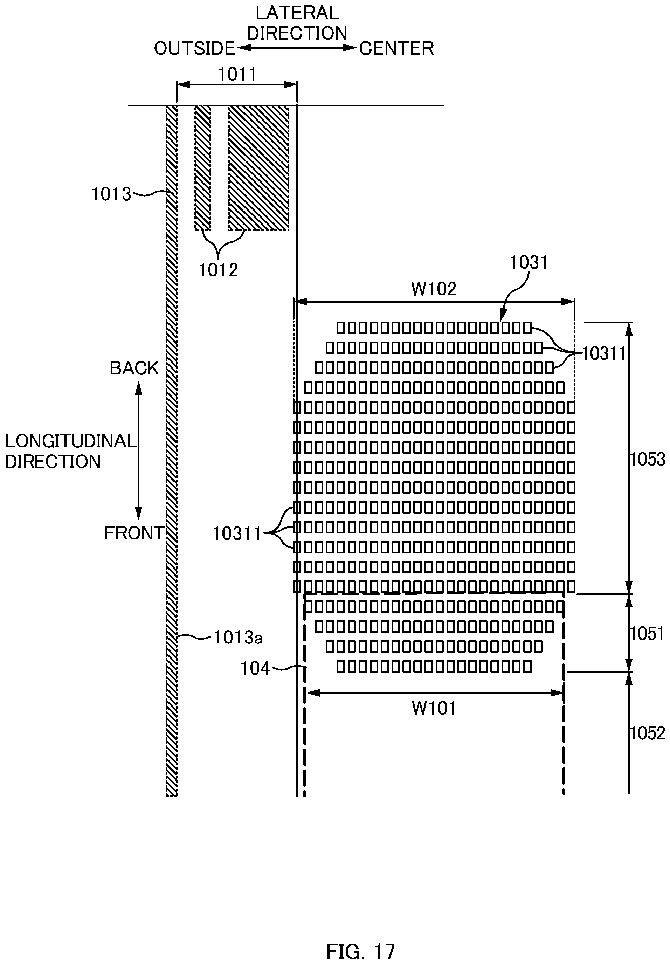

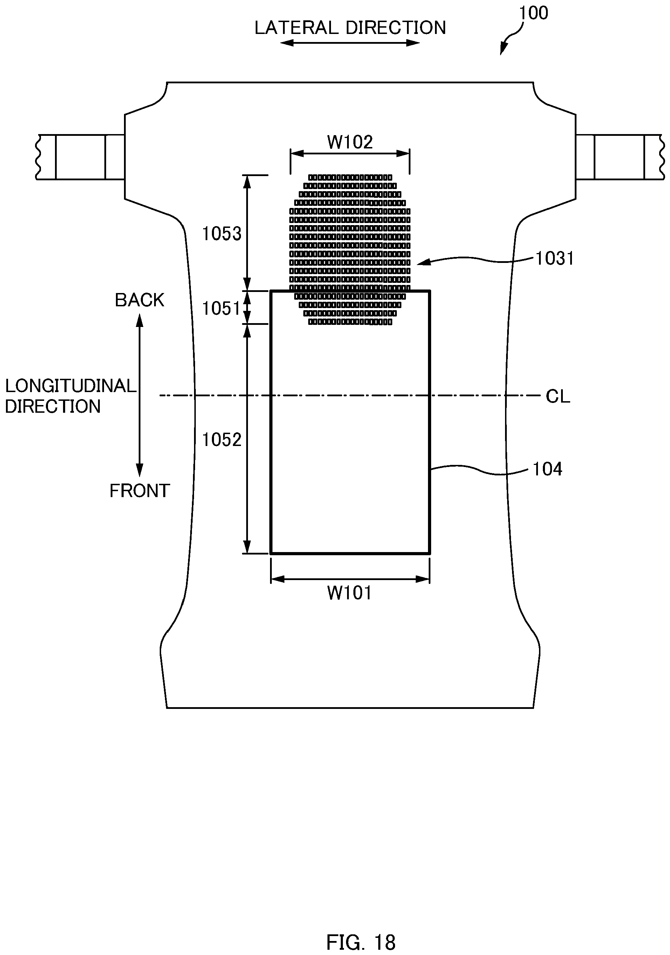

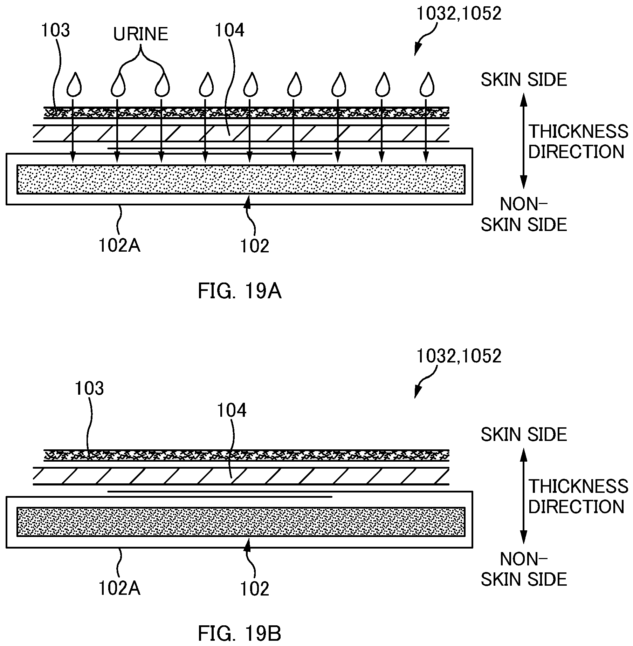

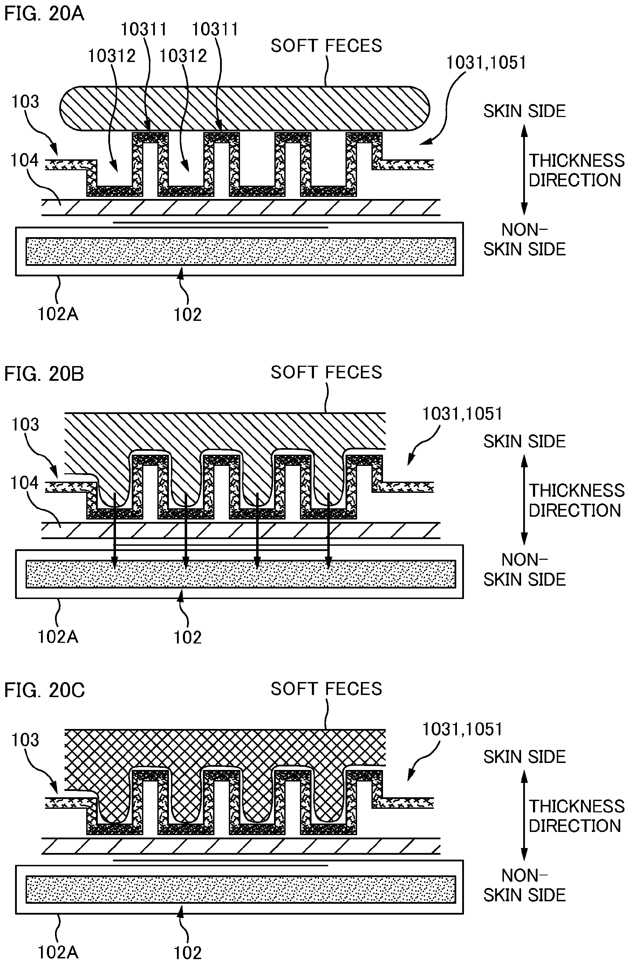

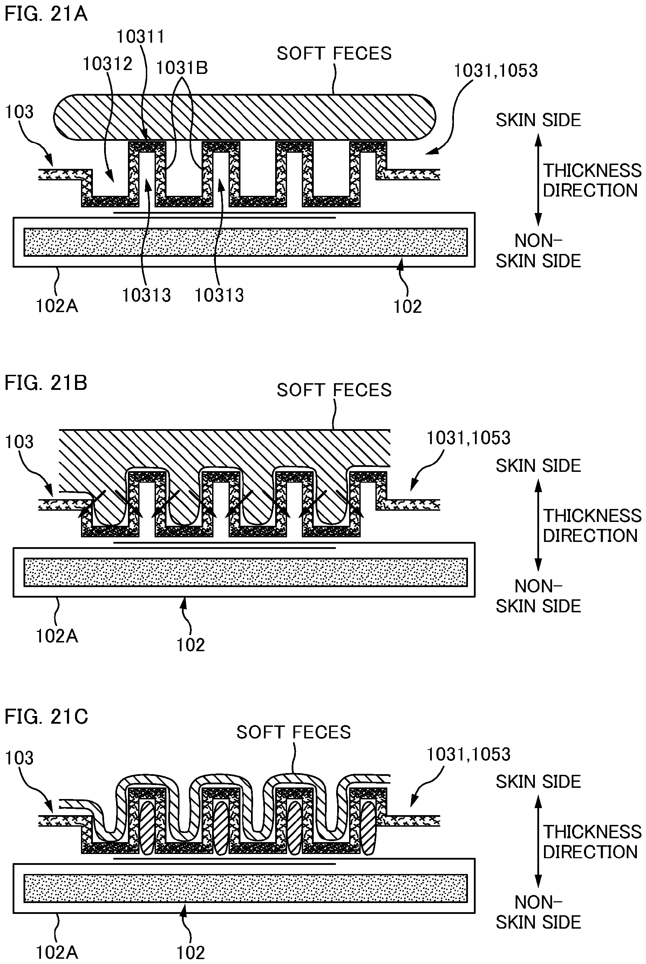

FIG. 16 is a schematic plan view of the diaper 100 manufactured by the manufacturing apparatus 200 of the diaper 100. FIG. 17 is a view showing the arrangement of the first region 1031 of the skin-side sheet 103 and the leak-proof wall portion 1011. FIG. 18 is a schematic plan view of the diaper 100 of a modified example. FIGS. 19A and 19B are explanatory diagrams illustrating how to absorb urine. FIGS. 20A to 20C and 21A to 21C are explanatory diagrams illustrating how to absorb soft feces.

The MD direction (the direction of transport of materials) in the manufacturing apparatus 200 of the diaper 100 corresponds to the longitudinal direction of the diaper 100, and the CD direction corresponds to the lateral direction of the diaper 100. In addition, the upstream side in the direction of transport of materials corresponds to the back side of the diaper 100 in the longitudinal direction, and the downstream side corresponds to the front side of the diaper 100 in the longitudinal direction.

The skin-side sheet 103 of the diaper 100 manufactured as described above includes: the first region 1031 which has protrusions and recesses by shaping into a protruded and recessed shape with the pair of shaping rolls 210 and 220; and the second region 1032 that is adjacent to the first region 1031 on the front side in the longitudinal direction and does not have protrusions and recesses shaped therein. In addition, the first region 1031 has a larger sparseness difference of fibers in the plane direction of the skin-side sheet 103 than the second region 1032. Furthermore, the skin-side sheet 103 has the second joining region 1052 and the first joining region 1051; in the second joining region 1052, one part of the intermediate sheet 104 is joined to the second region 1032, and in the first joining region 1051, another part of the intermediate sheet 104 is joined to the first region 1031.

According to such a diaper 100, during manufacturing, the intermediate sheet 104 can be first joined without any wrinkles to the second region 1032 which is likely to be transported in a tight state. In conjunction therewith, the intermediate sheet 104 can be joined without wrinkles to the first region 1031 in which protrusions and recesses are shaped in a tight state. Therefore, a wrinkle is not easily generated in the joining regions where the skin-side sheet 103 and the intermediate sheet 104 are joined, and the quality of the diaper 100 is enhanced.

In addition, in the intermediate sheet 104, protrusions and recesses are not shaped as in the skin-side sheet 103, and thus the skin-side surface of the intermediate sheet 104 is flat compared with the protruded and recessed shape of the first region 1031 of the skin-side sheet 103. Therefore, compared with a case where, for example, the intermediate sheet 104 is shaped into the protruded and recessed shape together with the skin-side sheet 103, the densities of the portions where fibers become dense due to the shaping of protrusions and recesses do not excessively increase, making it possible to decrease stiffness. Therefore, it is possible to soften the protrusion-and-recess-shaping portion (first region 1031) and to enhance the wearing comfortability of the diaper 100.

In addition, the second region 1032 (second joining region 1052) is positioned on the front side in the longitudinal direction, that is, in the urination region. In the second region 1032, the sparseness difference of fibers is small, and there is no portion where the density of fibers is high and urine is not easily permeated. Therefore, as shown in FIGS. 19A and 19B, in the second region 1032, it is possible to rapidly permeate urine throughout the entire region and to suppress urine leakage. In addition, in the second joining region 1052 in which the intermediate sheet 104 is disposed, the urine absorption property is enhanced, and additionally, it is also possible to suppress the backflow of urine.

On the other hand, the first region 1031 (first joining region 1051) is positioned on the back side in the longitudinal direction, that is, in the defecation region. The feces of an infant (particularly, an infant whose monthly age is relatively small), who is a target wearer of the diaper 100 of the present embodiment, is soft feces. Therefore, in the first region 1031 (the side wall portions 1031B shown in FIG. 13B), it becomes easy to take soft feces into the inside of the skin-side sheet 103 from the portions where fibers are sparse. Therefore, in the first region 1031, it is possible to reduce the amount of the soft feces that remains on the surface and to reduce the amount of soft feces attached to wearer.

In addition, in a case where the skin-side surface of the first region 1031 has a protruded and recessed shape, it is possible to suppress the flow of soft feces compared with a case where the skin-side surface is flat as in, for example, the second region 1032. This makes it possible to suppress the leakage of soft feces from the back side or leg circumferences.

The intermediate sheet 104 can be exemplified by a sheet that is coarser (have larger pores between fibers) than the absorbent body 102 (core-wrapping sheet 102A) and has a high liquid permeation rate (absorption rate). For example, the intermediate sheet 104 can be exemplified by a sheet containing air-through nonwoven fabric, spunbond nonwoven fabric, or crosslinked cellulose fiber and the like. The liquid permeation rates (absorption rates) can be compared by a well-known method. For example, a sample having the absorbent body 102 alone and a sample having the intermediate sheet 104 disposed on the absorbent body 102 are acquired. In addition, cylinders are placed on the samples, respectively, a predetermined amount of liquid is poured into each of the cylinders. And the periods of time taken from the time when the liquid begins to be poured to the time when the liquid in each cylinder has discharged are measured, thereby comparing the liquid permeation rates. Enlarged images of cross sections of the absorbent body 102 and the intermediate sheet 104 which are cut in the thickness direction are acquired, and in the enlarged images, the cross sections are each magnified at a magnification of approximately 20 to 100 times with an electron microscope or the like. In addition, using image analysis software, calculated are the area of pore portions per certain area in the cross section of the absorbent body 102 (core-wrapping sheet 102A) and the area of pore portions per certain area in the cross section of the intermediate sheet 104, comparing them.

In addition, the sparseness differences of fibers can be compared by a well-known method. For example, enlarged images of cross sections of the first region 1031 and the second region 1032 which are cut in the thickness direction are acquired, and in the enlarged images, the cross sections are each magnified at a magnification of approximately 20 to 100 times with an electron microscope or the like. In addition, in the cross section of the first region 1031, calculating the difference between the number of fibers per certain area of the portion where the fibers are dense (apex portion 1031A) and the number of fibers per certain area of the portion where the fibers are sparse (side wall portion 1031B), the difference is defined as the sparseness difference of fibers in the first region 1031. Similarly, in the cross section of the second region 1032, calculating the difference between the number of fibers per certain area of the portion where the fibers are dense and the number of fibers per certain area of the portion where the fibers are sparse, the difference is defined as the sparseness difference of fibers in the second region 1032. It is recommended that these sparseness differences of fibers are compared with each other. Alternatively, the sparseness differences of fibers may be compared visually from the enlarged cross-sectional images.

In addition, in the manufacturing apparatus 200 (FIG. 11), since the protruding portions 210A and 220A are provided on both outer circumferential surfaces of the pair of shaping rolls 210 and 220, the non-skin-side surface of the first region 1031 forms a protruded and recessed shape. Therefore, in the first region 1031, spaces 10313 are provided between the skin-side sheet 103 and the material adjacent to the skin-side sheet 103 on the non-skin side (the intermediate sheet 104 or the absorbent body 102). It is possible to take a larger amount of soft feces into these spaces 10313, making it possible to further reduce the amount of soft feces that remains on the skin-side sheet 103.

On the other hand, the non-skin-side surface of the second region 1032 in which protrusions and recesses are not shaped is flat compared with the non-skin-side surface of the first region 1031. Therefore, it becomes easy to securely join the flat surfaces of the second region 1032 and the intermediate sheet 104 without any wrinkles. In conjunction therewith, the first joining region 1051 is also easily joined without any wrinkles.

In addition, it is preferable that in a state where the diaper 100 is unfolded and stretched (FIG. 16), the longitudinal length (L102) of the second joining region 1052 is longer than the longitudinal length (L101) of the first joining region 1051 (L102 > L101). In such a case, the second joining region 1052, which is easily joined without any wrinkles while being transported in a tight state, can become long in the longitudinal direction, and in conjunction therewith, also making it easier to join the first joining region 1051 without any wrinkles. In addition, the second joining region 1052, which is suitable for urination, can be disposed across a broad range in the longitudinal direction. Therefore, it is possible to rapidly transfer urine, which has a higher fluidity than soft feces, to the intermediate sheet 104 in the longitudinally-large second joining region 1052, making it possible to suppress urine leakage.

In addition, it is preferable that in a state where the diaper 100 is unfolded and stretched (FIG. 16), the longitudinal center CL of the diaper 100 overlaps the second joining region 1052 in the longitudinal direction. In other words, it is preferable that the first region 1031 in which protrusions and recesses are shaped is positioned on the back side with respect to the longitudinal center CL of the diaper 100. In such a case, the second joining region 1052, which is suitable for urination, can be disposed on the front side with respect to the longitudinal center CL of the diaper 100, and the first region 1031, which is suitable for defecation, can be disposed on the back side with respect to the second joining region 1052. This makes it possible to reduce the amount of soft feces that remains in the first region 1031 while suppressing urine leakage.

In addition, in a packaged state, the diaper 100 is often folded in half in the longitudinal direction at the longitudinal center CL of the diaper 100 as shown in FIG. 15B. Accordingly, the second joining region 1052 is positioned at the longitudinal center CL of the diaper 100, making it less likely for the diaper 100 to be folded in the first region 1031 in which protrusions and recesses are shaped. Consequently, it is possible to suppress the collapse of the protrusions and recesses shaped. In addition, the intermediate sheet 104 is disposed in the second joining region 1052 and the second joining region 1052 is relatively highly stiff, making it possible to securely fold the diaper 100.

In addition, it is preferable that in the first region 1031, a plurality of protruding portions 10311 that protrude toward the skin side are arranged side-by-side at intervals in the lateral direction as shown in FIG. 17. For this purpose, it is recommended that the plurality of protruding portions 210A and 220A arranged in the CD direction are provided on the outer circumferential surfaces of the pair of shaping rolls 210 and 220 (FIG. 11). In such a case, longitudinal wrinkles (wrinkles that extend in the MD direction) that is likely to be generated in the first region 1031 of the skin-side sheet 103 being transported in the MD direction (the longitudinal direction of the diaper 100) can be divided by the protruding portions 210A and 220A of the shaping rolls 210 and 220. This makes it less likely to generate wrinkles in the first joining region 1051.

In addition, the plurality of protruding portions 10311 arranged in the lateral direction makes it possible to suppress the rate of soft feces that flows toward the back side in the longitudinal direction. Therefore, it is possible to suppress the leakage of soft feces from the back side.

Furthermore, it is preferable that the plurality of protruding portions 10311 arranged side-by-side in the lateral direction are arranged in the longitudinal direction. That is, it is preferable that the plurality of protruding portions 10311 are arranged at intervals in the longitudinal direction as well. In such a case, it is possible to suppress the rate of soft feces that flows in the lateral direction. Therefore, it is possible to suppress the leakage of soft feces from the leg circumference openings.

It should be noted that the protruded and recessed shape of the first region 1031 is not limited to the protruded and recessed shape shown in FIG. 17. For example, a plurality of laterally-elongated protruding portions in which the plurality of protruding portions 10311 arranged side-by-side in the lateral direction are integrated together may be arranged side-by-side in the longitudinal direction,. In addition, in FIG. 17, the lateral positions of the protruding portions 10311 which are adjacent to each other in the longitudinal direction are aligned, but may not be aligned.

In addition, it is preferable that in a state where the diaper 100 is unfolded and stretched (FIG. 16), the lateral length W101 of the intermediate sheet 104 is longer than half of the lateral length W102 of the first region 1031 (W101 > W102/2). In addition, as in a modified example of FIG. 18, in a state where the diaper 100 is unfolded and stretched, the lateral length W101 of the intermediate sheet 104 may be longer than the lateral length W102 of the first region 1031 (W101 > W102).

As described above, making long the lateral length W101 of the intermediate sheet 104 allows to make long in the lateral direction the second joining region 1052 which is easily joined without any wrinkles while being transported in a tight state. Therefore, in conjunction with the second joining region 1052, the first joining region 1051 is also more likely to be joined across a broad range in the lateral direction without any wrinkles. In addition, the second joining region 1052 which is suitable for urination is disposed long in the lateral direction, making it possible to rapidly transfer urine to the intermediate sheet 104 across a broad range in the lateral direction and making it possible to suppress urine leakage.

In addition, it is preferable that in a state where the diaper 100 is unfolded and stretched, as shown in FIG. 17, the lateral position of rising-up of the leak-proof wall portion 1011 (the lateral inner end 1013a of the longitudinal joining portion 1013) is positioned outside in the lateral direction with respect to the first region 1031. That is, it is preferable that the rising-up point of the leak-proof wall portion 1011 is not present in the first region 1031 that forms a protruded and recessed shape and that has a large sparseness difference of fibers. In such a case, the leak-proof wall portion 1011 stably rises up, making it possible for the leak-proof wall portion 1011 to dam soft feces.

In addition, the first region 1031 of the skin-side sheet 103 may have the non-joining region 1053 which is adjacent to the first joining region 1051 on the back side in the longitudinal direction and to which the intermediate sheet 104 is not joined.

As described above, the feces of an infant (particularly, an infant whose monthly age is relatively small), who is a target wearer of the diaper 100 of the present embodiment, is soft feces. Therefore, the inventors of the present application placed artificial feces that simulated soft feces on the first joining region 1051 and the non-joining region 1053 respectively, and observed them. In addition, after a predetermined period of time had elapsed, artificial skins were placed on the regions where the artificial feces had been placed, weights were placed on the artificial skins (placed were weights each of which was approximately as heavy as the body pressure of an infant whose wears the diaper). Then the amounts of the artificial feces attached to the artificial skins were observed.

As a result, the viscosity of the artificial feces that remained on the first joining region 1051 was higher than the viscosity of the artificial feces that remained on the non-joining region 1053. In addition, the amount (amount per certain area) of the artificial feces that remained on the first joining region 1051 was larger than the amount (amount per certain area) of the artificial feces that remained on the non-joining region 1053. Accordingly, the amount of the feces attached to the artificial skins placed on the first joining region 1051 was larger than the amount of the feces attached to the artificial skins placed on the non-joining region 1053.

The theory of the above-described phenomenon is considered as follows. In the first joining region 1051, as shown in FIGS. 20A to 20C, soft feces first enters recessed portions 10312 located between protruding portions 10311 that protrude on the skin side of the skin-side sheet 103. Then, moisture in the soft feces that has entered the recessed portions 10312 rapidly transfers to the intermediate sheet 104 that is in contact with the recessed portions 10312, increasing the viscosity of the soft feces. In addition, the permeation rate of the moisture in the soft feces is faster than the rate at which the entire soft feces is taken into the inside of the skin-side sheet 103. Accordingly, the viscosity of the soft feces increases before the entire soft feces is taken into the inside of the skin-side sheet 103. The soft feces having an increased viscosity is less likely to be taken into the inside of the skin-side sheet 103. Therefore, in the first joining region 1051, it can be considered that the viscosity of the soft feces remaining on the surface is high and that the amount of the remaining soft feces and the amount of the soft feces attached to the skin become relatively large.

On the other hand, in the non-joining region 1053, as shown in FIGS. 21A to 21C, the intermediate sheet 104 is not located below the skin-side sheet 103. Accordingly, there is no case where only the moisture in the soft feces that has entered the recessed portions 10312 of the skin-side sheet 103 transfers first. Consequently, the entire soft feces is taken into the inside of the skin-side sheet 103. In particular, since fibers are sparse in the side wall portions 1031B of the protrusions and recesses in the skin-side sheet 103, the soft feces is taken into spaces 10313 that are inside the protruding portions 10311, from the side wall portions 1031B. Therefore, in the non-joining region 1053, it can be considered that the viscosity of the soft feces remaining on the surface does not increase and that the amount of the remaining soft feces and the amount of the soft feces attached to the skin become small.

Therefore, as in the diaper 100 of the present embodiment, it is preferable that the non-joining region 1053 is provided on the back side of the first joining region 1051. Accordingly, firstly, moisture in soft feces excreted in the first joining region 1051 or soft feces that has flowed into the first joining region 1051 can be rapidly transferred to the intermediate sheet 104. Accordingly, it is possible to suppress the flow (rate) of the soft feces and to suppress the leakage of the soft feces from the back side. Thereafter, the soft feces that has transferred and flowed into the non-joining region 1053 on the back side, beyond the first joining region 1051 where the highly viscous soft feces remains, can be taken into the inside of the skin-side sheet 103. Therefore, it is possible to reduce the amount of the soft feces that remains in the skin-side sheet 103, making it possible to suppress the soft feces from being attached to the skin of a wearer.

Furthermore, it is preferable that in a state where the diaper 100 is unfolded and stretched (FIG. 16), the longitudinal length (L101) of the first joining region 1051 is shorter than the longitudinal length (L103) of the non-joining region 1053 (L101 < L103). In such a case, the non-joining region 1053, in which soft feces is easily taken into the inside of the skin-side sheet 103, can be disposed across a broad range in the longitudinal direction. This makes it possible to further reduce the amount of soft feces that remains on the skin-side sheet 103. In addition, the first joining region 1051, where there is a risk of the generation of wrinkles, becomes short in the longitudinal direction, making it less likely to generate wrinkles in the skin-side sheet 103.

In addition, it is preferable that in a state where the diaper 100 is unfolded and stretched (FIG. 16), the longitudinal length (L102) of the second joining region 1052 is longer than the longitudinal length (L103) of the non-joining region 1053 (L102 > L103). It is more preferable that, the longitudinal length (L102) of the second joining region 1052 is longer than the sum of the longitudinal length (L101) of the first joining region 1051 and the longitudinal length (L103) of the non-joining region 1053 (L102 > L101 + L103).

In such a case, the second joining region 1052, which is suitable for urination, can be disposed across a broad range in the longitudinal direction. This makes it possible to rapidly transfer urine to the intermediate sheet 104 in a broad range in the longitudinal direction, making it possible to suppress urine leakage. In addition, the second joining region 1052, which is easily joined without any wrinkles while being transported in a tight state, can become long in the longitudinal direction, and in conjunction therewith, also making it easier to join the first joining region 1051 without any wrinkles.

In addition, it is preferable that in a state where the diaper 100 is unfolded and stretched (FIG. 16), the longitudinal length (L101) of the first joining region 1051 is shorter than the length of half of the longitudinal length (product length L104) of the diaper 100 (L101 < L104/2). It is more preferable that, the sum of the longitudinal length (L101) of the first joining region 1051 and the longitudinal length (L103) of the non-joining region 1053 is shorter than the length of half of the product length (L104) of the diaper 100 (L101 + L103 < L104/2). In such a case, the first joining region 1051, where there is a risk of the generation of wrinkles, becomes short in the longitudinal direction, making it less likely to generate wrinkles in the skin-side sheet 103. In addition, the first joining region 1051 and the non-joining region 1053 can be disposed in the defecation region which is located on the back side with respect to the longitudinal center CL of the diaper 100.

In addition, in the protruding portions 10311 of the protruded and recessed shape formed in the first region 1031, the density of fibers is relatively high, and the stiffness is high. In the case of the tape-type diaper 100, portions that overlap the fastening tape 108 in the longitudinal direction are in close contact with the skin of a wearer more strongly. Therefore, it is preferable that in a state where the diaper 100 is unfolded and stretched, the first region 1031 is positioned on the front side in the longitudinal direction with respect to longitudinal centers 108a of the fastening tapes 108 as shown in FIG. 9. In such a case, it is possible to prevent for the fastening tapes 108 to make the highly stiff portions in the first region 1031 to be in close contact with the skin of a wearer strongly, making it possible to enhance wearing comfortability.

Similarly, in the case of the tape-type diaper 100, a portion that overlaps the waist stretchable member 109 in the longitudinal direction are in close contact with the skin of a wearer more strongly. Therefore, it is preferable that in a state where the diaper 100 is unfolded and stretched, the first region 1031 is positioned on the front side in the longitudinal direction with respect to the waist stretchable member 109 as shown in FIG. 9. In such a case, it is possible to prevent for the waist stretchable member 109 to make the highly stiff portions in the first region 1031 to be in close contact with the skin of a wearer strongly, making it possible to enhance wearing comfortability.

### Modified examples of skin-side sheet 103

### First modified example

The following configuration is acceptable: the first region 1031 has a protruded and recessed shape on either one of the skin-side surface and the non-skin-side surface, and the first region 1031 has a shape that is flat compared with the protruded and recessed shape, on the other one of the skin-side surface and the non-skin-side surface (not shown). Such a first region 1031 is formed with, for example, the pair of shaping rolls 210 and 220 in which protruding portions are provided on the outer circumferential surface of only either one of the pair of shaping rolls 210 and 220 and in which the outer circumferential surface of the other one of the pair of shaping rolls 210 and 220 is flat. In this case, the intermediate sheet 104 is first joined to the second region 1032 in which protrusions and recesses are not shaped, making it less likely to generate wrinkles in the joining region where the skin-side sheet 103 and the intermediate sheet 104 are joined.

### Second modified example

Hitherto, the second region 1032 has a flat shape, but the configuration is not limited thereto. The skin-side surface of the material that forms the skin-side sheet 103 may be made of a nonwoven fabric having a protruded and recessed shape (e.g., a nonwoven fabric having a protruded and recessed shape formed by blowing fluid to a fiber web), and the skin-side surface of the second region 1032 may have a protruded and recessed shape. Also in this case, the sparseness difference of fibers becomes small in the second region 1032 compared with the first region 1031 in which protrusions and recesses are shaped with the pair of shaping rolls 210 and 220. In addition, after protrusions and recesses are shaped in the first region 1031 and the second region 1032, additional protrusions and recesses may be shaped only in the first region 1031. Also in this case, the sparseness difference of fibers becomes small in the second region 1032 compared with the first region 1031. Therefore, in this case, the intermediate sheet 104 is first joined to the second region 1032, making it less likely to generate wrinkles in the joining region where the skin-side sheet 103 and the intermediate sheet 104 are joined.

### Third modified example

In the above embodiment, the non-joining region 1053 is provided on the back side of the first joining region 1051, but the configuration is not limited thereto. The following configuration is also acceptable: in the longitudinal direction of the diaper 100, the intermediate sheet 104 extend toward the back side beyond the back end of the first region 1031, the intermediate sheet 104 is joined to the entire longitudinal region of the first region 1031, and the non-joining region 1053 is not present.

Hitherto, the above embodiment has been described in order to facilitate the understanding of the present invention, not to interpret the present invention in a limited manner. The present disclosure may variously be changed or altered without departing from its gist and encompass equivalents thereof.

For example, the followings are not limited to the above-mentioned examples: the relationship between the lengths of the first joining region 1051, the second joining region 1052, the non-joining region 1053, and the intermediate sheet 104 in the longitudinal direction (direction of transport) or in the lateral direction, the positional relationship between the first region 1031 and the leak-proof wall portion 1011, and the like.

### [Reference Signs List]

1 Tape-type disposable diaper (absorbent article),
2 absorbent body, 3 skin-side sheet, 4 intermediate sheet,
5 leak-proof sheet, 6 exterior sheet, 7 side sheet,
8 fastening tape, 9 waist stretchable member,
10leg stretchable member, 11 leak-proof wall portion,
20, 21 shaping roll,
31 first surface region, 31A apex portion, 31B side wall portion,
32 second surface region,
33 first arrangement region (arrangement region), 34 first non-arrangement region (non-arrangement region),
35 second arrangement region,
100 tape-type disposable diaper (absorbent article),
102 absorbent body, 103 skin-side sheet, 104 intermediate sheet,
105 leak-proof sheet, 106 exterior sheet, 107 side sheet,
108 fastening tape, 109 waist stretchable member,
1010leg stretchable member, 1011 leak-proof wall portion,
210, 220 shaping roll (pair of rolls), 210A, 220A protruding portion,
1031 first region, 1031A apex portion, 1031B side wall portion,
10311 protruding portion, 10312 recessed portion, 10313 space,
1032 second region,
1051 first joining region, 1052 second joining region, 1053 non-joining region

## Claims

1. An absorbent article having a longitudinal direction and a lateral direction,
the absorbent article comprising:
an absorbent body;
a skin-side sheet located on a skin side with respect to the absorbent body; and
an intermediate sheet located between the absorbent body and the skin-side sheet,
the skin-side sheet having a first region and a second region,
the first region being a region that forms a protruded and recessed shape,
the second region being a region that is adjacent to the first region on a front side in the longitudinal direction,
the first region having a larger sparseness difference of fibers in a plane direction of the skin-side sheet than the second region has,
a skin-side surface of the intermediate sheet being flat compared with the protruded and recessed shape of the first region,
the skin-side sheet having a second joining region and a first joining region,
the second joining region being a region where one part of the intermediate sheet is joined to the second region,
the first joining region being a region where another part of the intermediate sheet is joined to the first region.

2. The absorbent article according to claim 1, wherein
a non-skin-side surface of the first region forms a protruded and recessed shape, and
a non-skin-side surface of the second region is flat compared with the non-skin-side surface of the first region.

3. The absorbent article according to claim 1 or 2, wherein
in a state where the absorbent article is unfolded and stretched,
a longitudinal length of the second joining region is longer than a longitudinal length of the first joining region.

4. The absorbent article according to any one of claims 1 to 3, wherein
the first region has a non-joining region that is adjacent to the first joining region on a back side in the longitudinal direction and to which the intermediate sheet is not joined, and
in a state where the absorbent article is unfolded and stretched,
a longitudinal length of the first joining region is shorter than a longitudinal length of the non-joining region.

5. The absorbent article according to any one of claims 1 to 4, wherein
the first region has a non-joining region that is adjacent to the first joining region on a back side in the longitudinal direction and to which the intermediate sheet is not joined, and
in a state where the absorbent article is unfolded and stretched,
a longitudinal length of the second joining region is longer than a longitudinal length of the non-joining region.

6. The absorbent article according to any one of claims 1 to 5, wherein
in a state where the absorbent article is unfolded and stretched,
a longitudinal center of the absorbent article overlaps the second joining region in the longitudinal direction.

7. The absorbent article according to any one of claims 1 to 6, wherein
in the first region, a plurality of protruding portions that protrude toward the skin side are arranged side-by-side in the lateral direction.

8. The absorbent article according to any one of claims 1 to 7, wherein
in a state where the absorbent article is unfolded and stretched,
a lateral length of the intermediate sheet is longer than half of a lateral length of the first region.

9. The absorbent article according to any one of claims 1 to 8, wherein
in a state where the absorbent article is unfolded and stretched,
a lateral length of the intermediate sheet is longer than a lateral length of the first region.

10. The absorbent article according to any one of claims 1 to 9, wherein
the absorbent article further comprises a pair of leak-proof wall portions that rise up toward the skin side, in two lateral side portions of the absorbent body, and
in a state where the absorbent article is unfolded and stretched,
a lateral position of rising-up of each of the leak-proof wall portions is positioned outside in the lateral direction with respect to the first region.
